# EUROPÄISCHE PATENTSCHRIFT

(11) **EP 1 628 980 B1**
(45) Veröffentlichungstag und Bekanntmachung des Hinweises auf die Patenterteilung: **28.04.2010**
(21) Anmeldenummer: 04739107.3
(22) Anmeldetag: 27.04.2004
(51) Int. Cl.: C07D 487/04, A61K 31/519, A61P 25/00

(54) **6-ARYLMETHYL-SUBSTITUIERTE PYRAZOLOPYRIMIDINE**
6-ARYLMETHYL-SUBSTITUTED PYRAZOLOPYRIMIDINES
PYRAZOLOPYRIMIDINES SUBSTITUEES PAR 6-ARYLMETHYLE

(30) Priorität: 09.05.2003 DE 10320785
(43) Veröffentlichungstag der Anmeldung: 01.03.2006
(73) Patentinhaber: Boehringer Ingelheim International GmbH, 55216 Ingelheim am Rhein (DE)
(72) Erfinder: HENDRIX, Martin, 51519 Odenthal (DE); BÄRFACKER, Lars, 46047 Oberhausen (DE); ERB, Christina, 65830 Kriftel (DE); HAFNER, Frank-Thorsten, 42115 Wuppertal (DE); HECKROTH, Heike, 42113 Wuppertal (DE); KARTHAUS , Dagmar, 42697 Solingen (DE); TERSTEEGEN, Adrian, 42115 Wuppertal (DE); VAN DER STAAY, Franz-Josef, CH-8251 BB Dronten (NL); VAN KAMPEN, Marja, 40221 Düsseldorf (DE)
(86) Internationale Anmeldenummer: PCT/EP2004/004412
(87) Internationale Veröffentlichungsnummer: WO 2004/099210

(56) Entgegenhaltungen:
- WO-A-02/055082
- WO-A-02/068423
- WO-A-03/037899
- WO-A-2004/018474
- WO-A-2004/026286
- WO-A-2004/026876

## Beschreibung

Die Erfindung betrifft neue 6-Arylmethyl-substituierte Pyrazolopyrimidine, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Inhibition von Phosphordiesterasen moduliert die Spiegel der zyklischen Nukleotide 5'-3' zyklisches Adenosinmonophosphat (cAMP) bzw. 5'-3' zyklisches Guanosinmonophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Proteinkinasen. Die von cAMP aktivierte Proteinkinase wird Proteinkinase A (PKA) genannt, die von cGMP aktivierte Proteinkinase wird Proteinkinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., Prog. Neurobiol., 1998, 56: 37 - 64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE Gene beschrieben (Exp. Opin. Investig. Drugs 2000, 9, 1354-3784). Diese 21 PDE Gene lassen sich aufgrund ihrer Sequenzhomologie in 11 PDE Familien einteilen (Nomenklatur Vorschlag siehe http://depts.washington.edu/pde/Nomenclature.html.). Einzelne PDE Gene innerhalb einer Familie werden durch Buchstaben unterschieden (z.B. PDE1A und PDE1B). Falls noch unterschiedliche Splice Varianten innerhalb eines Genes vorkommen, wird dies dann durch eine zusätzliche Nummerierung nach dem Buchstaben angegeben (z.B. PDE1A1).

Die Humane PDE9A wurde 1998 kloniert und sequenziert. Die Aminosäurenidentität zu anderen PDEs liegt bei maximal 34 % (PDE8A) und minimal 28 % (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP Bindungsdomäne auf, die auf eine allosterische Enzymregulation durch cGMP schließen ließe. In einer Western Blot Analyse wurde gezeigt, dass die PDE9A im Mensch unter anderem in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., J. Biol. Chem., 1998, 273 (25): 15559 - 15564). Das Gen für die humane PDE9A liegt auf Chromosom 21q22.3 und enthält 21. Exons. Bislang wurden 4 alternative Spleißvarianten der PDE9A identifiziert (Guipponi et al., Hum. Genet., 1998, 103: 386 - 392), Klassische PDE Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., J. Biol. Chem., 1998, 273 (25): 15559 - 15564).

Die Maus PDE9A wurde 1998 von Soderling et al. (J. Biol. Chem., 1998, 273 (19): 15553 - 15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus PDE9A wird von IBMX in Konzentrationen unter 200 µM nicht gehemmt; der IC₅₀-Wert für Zaprinast liegt bei 29 µM (Soderling et al., J. Biol. Chem., 1998, 273 (19): 15553 - 15558). Im Rattengehirn wurde gezeigt, dass PDE9A in einigen Hirnregionen stark exprimiert wird. Dazu zählen der Bulbus olfactorius, Hippocampus, Cortex, Basalganglien und basales Vorderhirn (Andreeva et al., J. Neurosci., 2001, 21 (22): 9068 - 9076). Insbesondere Hippocampus, Cortex und basales Vorderhirn spielen eine wichtige Rolle an Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., J. Biol. Chem., 1982, 257: 1973 - 1979), PDESA (Km = 4 µM; Francis et al., J. Biol. Chem., 1980, 255: 620 - 626), PDE6A (Km = 17 µM; Gillespie and Beavo, J. Biol. Chem., 1988, 263 (17): 8133 - 8141) und PDE11A (Km = 0,52 µM; Fawcett et al., Proc. Nat. Acad. Sci., 2000, 97 (7): 3702 - 3707) schon bei niedrigen physiologischen Konzentrationen aktiv. Im Gegensatz zu PDE2A (Murashima et al., Biochemistry, 1990, 29: 5285 - 5292) wird die katalytische Aktvität von PDE9A nicht durch cGMP gesteigert, da es keine GAF Domäne (cGMP Bindedomäne, über die die PDE Aktivität allosterisch gesteigert wird) aufweist (Beavo et al., Current Opinion in Cell Biology, 2000,12: 174 -179). PDE9A Inhibitoren können deshalb zu einer Erhöhung der basalen cGMP Konzentration führen.

Die WO 98/40384 offenbart Pyrazolopyrimidine, die sich als PDE1-, 2- und 5-Inhibitoren auszeichnen und für die Behandlung von cardiovascularen, cerebrovascularen Erkrankungen sowie Erkrankungen des Urogenitalbereiches eingesetzt werden können.

In CH 396 924, CH 396 925, CH 396 926, CH 396 927, DE 1 147 234, DE 1 149 013, GB 937,726 werden Pyrazolopyrimidine mit coronarerweiternder Wirkung beschrieben, die zur Behandlung von Durchblutungsstörungen des Herzmuskels eingesetzt werden können.

Im US 3,732,225 werden Pyrazolopyrimidine beschrieben, die eine entzündungshemmende und Blutzucker-senkende Wirkung haben.

In DE 2 408 906 werden Styrolpyrazolopyrimidine beschrieben, die als antimikrobielle und entzündungshemmende Mittel für die Behandlung von beispielsweise Ödem eingesetzt werden können.

Die vorliegende Erfindung betrifft Verbindungen der Formel 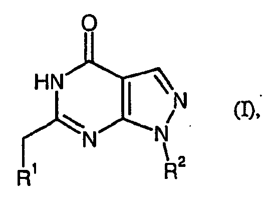 in welcher
- R¹: Phenyl, Pyridyl oder Thiophenyl, welche gegebenenfalls mit bis zu 3 Substituenten un- abhängig voneinander ausgewählt aus der Gruppe C₁-C₆-Alkyl, C₁-C₆-Alkoxy, Hydroxy- carbonyl, Cyano, Trifluormethyl, Amino, Nitro, Hydroxy, C₁-C₆-Alkylamino, Halogen, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonyl- amino, C₁-C₆-Alkylsulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxy- carbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8-gliedriges Heterocyclyl bedeuten, substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆- Alkyl, C₁-C₆-Alkoxy, Hydroxycarbonyl, Cyano, Trifluormethyl, Amino, Nitro, Hydroxy, C₁-C₆-Alkylamino, Halogen, C₆-C₁₀-Arylcarbonylamino, C₁-C₆-Alkylcarbonylamino, C₁-C₆-Alkylaminocarbonyl, C₁-C₆-Alkoxycarbonyl, C₆-C₁₀-Arylaminocarbonyl, Hetero- arylaminocarbonyl, Heteroarylcarbonylamino, C₁-C₆-Alkylsulfonylamino, C₁-C₆-Alkyl- sulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆- Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxy- carbonyl und einer Gruppe der Formel -NR³R⁴,
wobei R³ und R⁴ die oben angegebenen Bedeutungen aufweisen, substituiert sind,
bedeuten, sowie deren Salze, Solvate und/oder Solvate der Salze.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dehydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der. Solvate, bei denen die Koordination mit Wasser erfolgt.

Im Rahmen der vorliegenden Erfindung haben die Substituenten, soweit nicht anders spezifiziert, die folgende Bedeutung:

C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Bevorzugte Beispiele umfassen Methoxy, Ethoxy, n-Propoxy, Isopropoxy, tert.-Butoxy, n-Pentoxy und n-Hexoxy.

C₁-C₆-Alkyl steht für einen geradkettigen oder verzweigten Alkylrest mit 1 bis 6, bevorzugt 1 bis 4, besonders bevorzugt 1 bis 3 Kohlenstoffatomen. Bevorzugte Beispiele umfassen Methyl, Ethyl, n-Propyl, Isopropyl, tert.-Butyl, n-Pentyl und n-Hexyl.

Halogen steht für Fluor, Chlor, Brom und Iod. Bevorzugt sind Fluor, Chlor, Brom, besonders bevorzugt Fluor und Chlor.

C₁-C₆-Alkylamino steht für einen geradkettigen oder verzweigten Mono- oder Dialkylaminorest mit 1 bis 6, bevorzugt 1 bis 4 und besonders bevorzugt mit 1 bis 3 Kohlenstoffatomen. Bevorzugte Beispiele umfassen Methylamino, Ethylamino, n-Propylamino, Isopropylamino, tert.Butylamino, n-Pentylamino und n-Hexylamino, Dimethylamino, Diethylamino, Di-n-propylamino, Diisopropylamino, Di-t-butylamino, Di-n-pentylamino, Di-n-hexylamino, Ethylmethylamino, Isopropylmethylamino, n-Butylethylamino und n-Hexyl-i-pentylamino.

C₁-C₆-Alkylcarbonylamino steht für einen über eine Amino-Gruppe verknüpften Alkylcarbonylrest, wobei der Alkylrest geradkettig oder verzweigt sein kann und 1 bis 6, bevorzugt 1 bis 4, und besonders bevorzugt 1 bis 3 Kohlenstoffatome enthält. Bevorzugte Beispiele umfassen Methylcarbonylamino, Ethylcarbonylamino, n-Propylcarbonylamino, Isopropylcarbonylamino, tert.Butylcarbonylamino, n-Pentylcarbonylamino und n-Hexylcarbonylamino.

C₁-C₆-Alkylaminocarbonyl steht für einen über eine Carbonyl-Gruppe verknüpften Mono- oder Dialkylaminorest, wobei die Alkylreste gleich oder verschieden sein können, geradkettig oder verzweigt sind und jeweils 1 bis 6, bevorzugt 1 bis 4, und besonders bevorzugt 1 bis 3 Kohlenstoffatome enthalten. Bevorzugte Beispiele umfassen Methylaminocarbonyl, Ethylaminocarbonyl, n-Propylaminocarbonyl, Isopropylaminocarbonyl, tert.Butylaminocarbonyl, n-Pentylaminocarbonyl, n-Hexylaminocarbonyl, Dimethylaminocarbonyl, Diethylaminocarbonyl, Di-n-propylaminocarbonyl, Diisopropylaminocarbonyl, Di-t-butylanzinocarbonyl, Di-n-pentylaminocarbonyl, Di-n-hexylaminocarbonyl, Ethylmethylaminocarbonyl, Isopropylmethylaminocarbonyl, n-Butylethylaminocarbonyl und n-Hexyl-i-pentylaminocarbonyl. Weiterhin können im Falle eines Diaklaminorestes die beiden Alkylreste zusammen mit dem Stickstoffatom, an das sie gebunden sind, einen 5- bis 8-gliedriges Heterocyclyl bilden.

C₆-C₁₀-Arylaminocarbonyl steht für einen über eine Carbonyl-Gruppe verknüpften Arylaminorest. Bevorzugte Beispiele umfassen Phenylaminocarbonyl und Naphthylaminocarbonyl.

C₆-C₁₀-Arylcarbonylamino steht für einen über eine Amino-Gruppe verknüpften Arylcarbonylrest. Bevorzugte Beispiele umfassen Phenylcarbonylamino und Naphthylcarbonylamino.

C₁-C₆-Alkylsulfonylamino steht für einen geradkettigen oder verzweigten Alkylsulfonylaminorest mit 1 bis 6, bevorzugt 1 bis 4 und besonders bevorzugt mit 1 bis 3 Kohlenstoffatomen. Bevorzugte Beispiele umfassen Methylsulfonylamino, Ethylsulfonylamino, n-Propylsulfonylamino, Isopropylsulfonylamino, tert.Butylsulfonylamino, n-Pentylsulfonylamino und n-Hexylsulfonylamino.

C₁-C₆-Alkylsulfonyl steht für einen geradkettigen oder verzweigten Alkylsulfonylrest mit 1 bis 6, bevorzugt 1 bis 4 und besonders bevorzugt mit 1 bis 3 Kohlenstoffatomen. Bevorzugte Beispiele umfassen Methylsulfonyl, Ethylsulfonyl, n-Propylsulfonyl, Isopropylsulfonyl, tert.Butylsulfonyl, n-Pentylsulfonyl und n-Hexylsulfonyl.

C₁-C₆-Alkylthio steht für einen geradkettigen oder verzweigten Alkylthiorest mit 1 bis 6, bevorzugt 1 bis 4 und besonders bevorzugt mit 1 bis 3 Kohlenstoffatomen. Bevorzugte Beispiele umfassen Methylthio, Ethylthio, n-Propylthio, Isopropylthio, tert.Butylthio, n-Pentylthio und n-Hexylthio.

Heteroaryl steht für einen aromatischen, mono- oder bicyclischen Rest mit 5 bis 10 Ringatomen und bis zu 5 Heteroatomen aus der Reihe S, O und/oder N. Bevorzugt sind 5- bis 6-gliedrige Heteroaryle mit bis zu 4 Heteroatomen. Der Heteroarylrest kann über ein Kohlenstoff- oder Stickstoffatom gebunden sein. Bevorzugte Beispiele umfassen Thienyl, Furyl, Pyrrolyl, Thiazolyl, Oxazolyl, Imidazolyl, Tetrazolyl, Pyridyl, Pyrimidinyl, Pyridazinyl, Indolyl, Indazolyl, Benzofuranyl, Benzothiophenyl, Chinolinyl und Isochinolinyl.

Heteroarylaminocarbonyl steht für einen über eine Carbonyl-Gruppe verknüpften Heteroarylamino-Rest. Bevorzugte Beispiele umfassen Thienylaminocarbonyl, Furylaminocarbonyl, Pyrrolylaminocarbonyl, Thiazolylaminocarbonyl, Oxazolylaminocarbonyl, Imidazolylaminocarbonyl, Tetrazolylaminocarbonyl, Pyridylaminocarbonyl, Pyrimidinylaminocarbonyl, Pyridazinylaminocarbonyl, Indolylaminocarbonyl, Indazolylaminocarbonyl, Benzofuranylaminocarbonyl, Benzothiophenylaminocarbonyl, Chinolinylaminocarbonyl und Isochinolinylaminocarbonyl.

Heteroarylcarbonylamino steht für einen über eine Amino-Gruppe verknüpften Heteroarylcarbonyl-Rest. Bevorzugte Beispiele umfassen Thienylcarbonylamino, Furylcarbonylamino, Pyrrolylcarbonylamino, Thiazolylcarbonylamino, Oxazolylcarbonylamino, Imidazolylcarbonylamino, Tetrazolylcarbonylamino, Pyridylcarbonylamino, Pyrimidinylcarbonylamino, Pyridazinylcarbonylamino, Indolylcarbonylamino, Indazolylcarbonylamino, Benzofuranylcarbonylamino, Benzothiophenylcarbonylamino, Chinolinylcarbonylamino und Isochinolinylcarbonylamino.

5- bis 8-gliedriges Heterocyclyl steht für einen mono- oder polycyclischen, heterocyclischen Rest mit 5 bis 8 Ringatomen und bis zu 3, vorzugsweise 2 Heteroatomen bzw. Heterogruppen aus der Reihe N, O, S, SO, SO₂. Mono- oder bicyclisches Heterocyclyl ist bevorzugt. Besonders bevorzugt ist monocyclisches Heterocyclyl. Als Heteroatome sind N und O bevorzugt. Die Heterocyclyl-Reste können gesättigt oder teilweise ungesättigt sein. Gesättigte Heterocyclyl-Reste sind bevorzugt. Besonders bevorzugt sind 5- bis 7-gliedrige Heterocyclylreste. Bevorzugte Beispiele umfassen Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl.

6-gliedriges Heteroaryl steht für einen aromatischen Rest mit 6 Ringatomen und bis zu 2 Stickstoffatomen. Der Heteroarylrest ist über ein Kohlenstoffatom gebunden. Bevorzugte Beispiele umfassen Pyridyl, Pyrimidinyl, Pyridazinyl und Pyrazinyl.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Die erfindungsgemäßen Verbindungen können auch als Tautomere vorliegen, wie im Folgenden beispielhaft gezeigt wird: 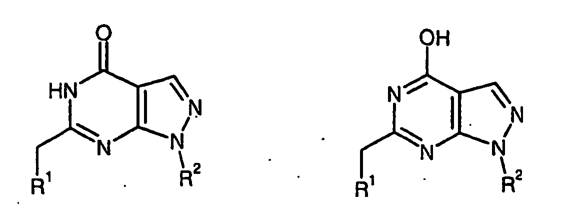 Eine weitere Ausführungsform der Erfindung betrifft Verbindungen der Formel (I),
in welcher
- R¹: Phenyl, Pyridyl oder Thiophenyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀- Arylcarbonylamino, C₁-C₄-Alkylcarbonylamino, C₁-C₄-Alkylaminocarbonyl, C₁-C₄- Alkoxycarbonyl, C₆-C₁₀-Arylaminocarbonyl, Heteroarylaminocarbonyl, Heteroaryl- carbonylamino, C₁-C₄-Alkylulfonylamino, C₁-C₄-Alkylsulfonyl, C₁-C₄-Alkylthio substituiert sind,
wobei C₁-C₄-Alkyl und C₁-C₄-Akoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ unabhängig voneinander Wasserstoff oder C₁-C₄-Alkyl, oder
R³ und R⁴ zusammen mit dem Stickstoffatom, an das sie gebunden sind, 5- bis 6-gliedriges Heterocyclyl bedeuten, substituiert sind,
- R²: Phenyl, Pyrimidyl oder Pyridyl, wobei Phenyl mit 1 bis 3 Resten und Pyrimidyl und Pyridyl gegebenenfalls mit 1 bis 3 Resten jeweils unabhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkyl- carbonylamino, C₁-C₄-Alkylaminocarbonyl, C₁-C₄-Alkoxycarbonyl, C₆-C₁₀-Arylamino- carbonyl, Heteroarylaminocarbonyl, Heteroarylcarbonylamino, C₁-C₄-Alkylsulfonylamino, C₁-C₄-Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴, wobei R³ und R⁴ die oben angegebenen Bedeutungen aufweisen, substituiert sind,
bedeuten, sowie deren Salze, Solvate und/oder Solvate der Salze.

Eine weitere Ausführungsform der Erfindung betrifft Verbindungen der Formel (I),
in welcher R¹ die oben angegebenen Bedeutungen aufweist und
- R²: Phenyl oder Pyridyl, wobei Phenyl mit 1 bis 2 Resten und Pyridyl gegebenenfalls mit 1 bis 2 Resten jeweils unabhängig voneinander ausgewählt aus der Gruppe Methyl, Ethyl, 2- Propyl, Trifluormethyl, Methoxy, Ethoxy, Fluor und Chlor substituiert sind,
bedeutet, sowie deren Salze, Solvate und/oder Solvate der Salze.

Eine weitere Ausführungsform der Erfindung betrifft Verbindungen der Formel (I),
in welcher
- R¹: Phenyl, Pyridyl oder Thiophenyl, welche gegebenenfalls mit bis zu 2 Resten unabhängig voneinander ausgewählt aus der Gruppe C₁-C₄-Alkyl, Fluor, Chlor, Trifluormethyl, Hydroxy, Phenylcarbonylamino, C₁-C₄-Alkylcarbonylamino, C₁-C₄-Alkylaminocarbonyl oder Phenylaminocarbonyl substituiert sind,
- R²: Phenyl oder Pyridyl, wobei Phenyl mit 1 bis 2 Resten und Pyridyl gegebenenfalls mit 1 bis 2 Resten jeweils unabhängig voneinander ausgewählt aus der Gruppe Methyl, Ethyl, 2- Propyl, Trifluormethyl, Methoxy, Ethoxy, Fluor und Chlor substituiert sind,
bedeuten, sowie deren Salze, Solvate und/oder Solvate der Salze.

Außerdem wurde ein Verfahren zur Herstellung der erfindungsgemäßen Verbindungen der Formel (I) gefunden, **dadurch gekennzeichnet, dass** man entweder

### [A] Verbindungen der Formel

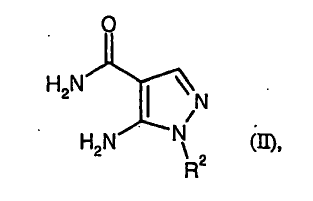

in welcher
- R³: die oben angegebenen Bedeutungen hat,
durch Umsetzung mit einer Verbindung der Formel

R¹-CH₂-C(O)-Z (IIIa),

in welcher
- R¹: die oben angegebenen Bedeutungen hat,
und
- Z: für Chlor oder Brom steht,
in einem inerten Lösemittel und in Anwesenheit einer Base zunächst in Verbindungen der Formel 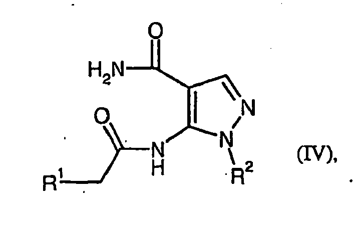 in welcher
- R¹ und R²: die oben angegebenen Bedeutungen haben,
überführt, dann in einem inerten Lösemittel in Gegenwart einer Base zu Verbindungen der Formel (I) cyclisiert,
oder

### [B] Verbindungen der Formel (II) unter direkter Cyclisierung zu (I) mit einer Verbindung der Formel

R¹-CH₂-C(O)-OR³ (IIIb),

in welcher
- R¹: die oben angegebenen Bedeutungen hat,
und
- R³: für Methyl oder Ethyl steht,
in einem inerten Lösemittel und in Anwesenheit einer Base umsetzt,
oder

### [C] Verbindungen der Formel

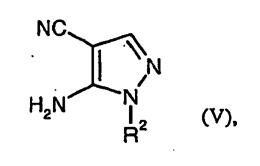

in welcher
- R²: die oben angegebenen Bedeutungen hat,
zunächst durch Umsetzung mit einer Verbindung der Formel (IIIa) in einem inerten Lösemittel und in Anwesenheit einer Base in Verbindungen der Formel 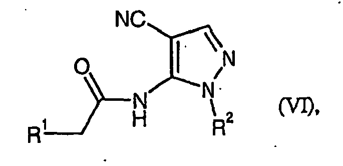 in welcher
- R¹ und R²: die oben angegebenen Bedeutungen haben,
überführt,
und diese in einem zweiten Schritt in einem inerten Lösemittel und in Anwesenheit einer Base und eines Oxidationsmittels zu (I) cyclisiert,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Für den ersten Schritt des Verfahrens [A] und des Verfahrens [C] eignen sich inerte organische Lösemittel, die sich unter den Reaktionsbedingungen nicht verändern. Hierzu gehören bevorzugt Ether wie beispielsweise Diethylether. Dioxan, Tetrahydrofuran oder Glykoldimethylether, oder Toluol oder Pyridin. Ebenso ist es möglich, Gemische der genannten Lösemittel einzusetzen. Besonders bevorzugt sind Tetrahydrofuran, Toluol oder Pyridin.

Als Basen eignen sich im Allgemeinen Alkalihydride, wie beispielsweise Natriumhydrid, oder cyclische Amine, wie beispielsweise Piperidin, Pyridin, Dimethylaminopyridin (DMAP), oder C₁-C₄-Alkylamine, wie beispielsweise Triethylamin. Bevorzugt sind Natriumhydrid, Pyridin und/oder Dimethylaminopyridin.

Die Base wird im Allgemeinen in einer Menge von 1 mol bis 4 mol, bevorzugt von 1,2 mol bis 3 mol, jeweils bezogen auf 1 mol der Verbindungen der Formel (II) bzw. (V), eingesetzt In einer Variante wird die Umsetzung in Pyridin, dem eine katalytische Menge DMAP zugesetzt wird, durchgeführt. Gegebenenfalls kann noch Toluol zugefügt werden.

Die Reaktionstemperatur kann im Allgemeinen in einem größeren Bereich variiert werden. Im Allgemeinen arbeitet man in einem Bereich von -20°C bis +200°C, bevorzugt von 0°C bis +100°C.

Als Lösemittel für die Cyclisierung im zweiten Schritt der Verfahren [A] und [C] eignen sich die üblichen organischen Lösemittel. Hierzu gehören bevorzugt Alkohole wie Methanol, Ethanol, Propanol, Isopropanol, n-Butanol oder tert.-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol oder tert.-Butanol verwendet. Ebenso ist es möglich, Gemische der genannten Lösemittel einzusetzen.

Als Basen für die Cyclisierung im zweiten Schritt der Verfahren [A] und [C] eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.-butanolat. Besonders bevorzugt sind Kaliumcarbonat, Natriumhydroxid und Kalium-tert.-butanolat.

Bei der Durchführung der Cyclisierung wird die Base im Allgemeinen in einer Menge von 2 mol bis 6 mol, bevorzugt von 3 mol bis 5 mol, jeweils bezogen auf 1 mol der Verbindungen der Formel (IV) bzw. (VI), eingesetzt.

Als Oxidationsmittel für die Cyclisierung im zweiten Schritt des Verfahrens [C] eignen sich beispielsweise Wasserstoffperoxid oder Natriumborat. Bevorzugt ist Wasserstoffperoxid.

Die Cyclisierung in den Verfahren [A], [B] und [C] wird im Allgemeinen in einem Temperaturbereich von 0°C bis +160°C, bevorzugt bei der Siedetemperatur des jeweiligen Lösemittels durchgeführt.

Die Cyclisierung wird im Allgemeinen bei Normaldruck durchgeführt. Es ist aber auch möglich, das Verfahren bei Überdruck oder bei Unterdruck durchzuführen (z.B. in einem Bereich von 0.5 bis 5 bar).

Als Lösemittel für das Verfahren [B] eignen sich die oben für den zweiten Schritt der Verfahren [A] und [C] aufgeführten Alkohole, wobei Ethanol bevorzugt ist.

Als Basen für das Verfahren [B] eignen sich Alkalihydride, wie beispielsweise Natrium- oder Kaliumhydrid, oder Alkalialkoholate, wie beispielsweise Natriummethanolat, -ethanolat, -isopropylat oder Kalium-tert.-butylat. Bevorzugt ist Natriumhydrid.

Die Base wird in einer Menge von 2 mol bis 8 mol, bevorzugt von 3 mol bis 6 mol, jeweils bezogen auf 1 mol der Verbindungen der Formel (II), eingesetzt.

Die Verbindungen der Formel (II) sind bekannt oder können beispielsweise hergestellt werden, indem man zunächst Ethoxymethylenmalonsäuredinitril mit Hydrazin-Derivaten der Formel

R²-NH-NH₂ (VII),

in welcher
- R²: die oben angegebenen Bedeutungen hat,
in einem inerten Lösemittel zu den Pyrazolnitrilen der Formel (V) kondensiert und diese dann mit einem der oben aufgeführten Oxidationsmittel, vorzugsweise Wasserstoffperoxid, in Anwesenheit von Ammoniak umsetzt [vgl. z.B. A. Miyashita et al., Heterocycles 1990, 31, 1309ff].

Die Verbindungen der Formeln (IIa), (IIIb) und (VII) sind kommerziell erhältlich, literaturbekannt oder können in Analogie zu literaturbekannten Verfahren hergestellt werden.

Das erfindungsgemäße Verfahren kann durch das folgendes Formelschema beispielhaft erläutert werden:

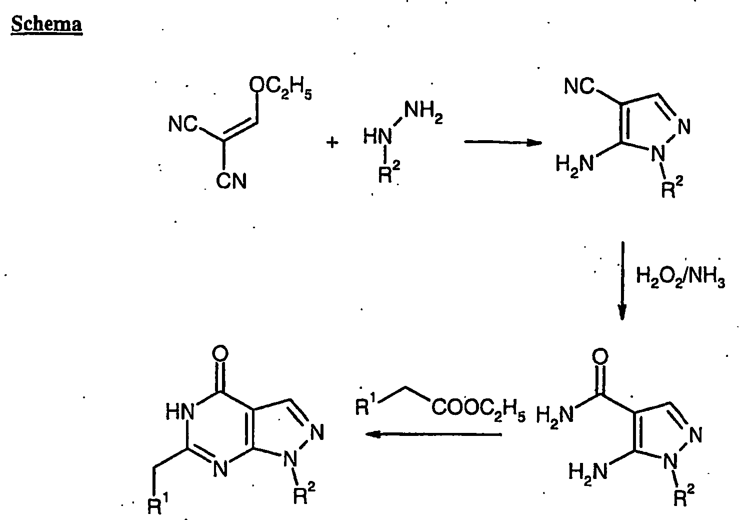

Weitere Verfahren zur Herstellung von Pyrazolo[3,4-d]pyrimidin-4-onen sind bekannt und können ebenfalls zur Synthese der erfindungsgemäßen Verbindungen eingesetzt werden (siehe zum Beispiel: P. Schmidt et al., *Helvetica Chimica Acta* **1962**, *189*, 1620ff.).

Die erfindungsgemäßen Verbindungen zeigen ein nicht vorhersehbares, wertvolles pharmakologisches Wirkspektrum. Sie zeichnen sich insbesondere durch eine Inhibition von PDE9A aus.

Überraschenderweise wurde gefunden, dass die erfindungsgemäßen Verbindungen zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen Eigenschaften allein oder in Kombination mit anderen Arzneimitteln zur Verbesserung von Wahrnehmung, Konzentrationsleistung, Lern- und/oder Gedächtnisleistung eingesetzt werden.

Besonders eignen sich die erfindungsgemäßen Verbindungen zur Verbesserung der Wahrnehmung, Konzentrationsleistung, Lernleistung, oder Gedächthisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Him-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die *in vitro*-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. J. Biol. Chem. 1996 271, 796-806), PDE2A (GenBank/EMBL Accession Number: NM_002599, Rosman et al. Gene 1997 191, 89-95), PDE3B (GenBank/EMBL Accession Number: NM_000922, Miki et al. Genomics 1996, 36, 476-485), PDE4B (GenBank/EMBL Accession Number: NM_002600, Obernolte et al. Gene. 1993, 129, 239-247), PDE5A (GenBank/EMBL Accession Number: NM_001083, Loughney et al. Gene 1998, 216, 139-147), PDE7B (GenBank/EMBL Accession Number: NM_018945, Hetman et al. Proc. Natl. Acad. Sci. U.S.A. 2000, 97, 472-476), PDE8A (GenBank/EMBL Accession Number: AF_056490, Fisher et al. Biochem. Biophys. Res. Commun. 1998 246, 570-577), PDE9A (Fisher et al., J. Biol. Chem, 1998, 273 (25): 15559 - 15564), E10A (GenBank/EMBL Accession Number: NM_06661, Fujishige et al. J Biol Chem. 1999, 274, 18438-45.), PDE11A (GenBank/EMBL Accession Number: NM_016953, Fawcett et al. Proc. Natl. Acad. Sci. 2000, 97, 3702-3707) wurden mit Hilfe des pFASTBAC Baculovirus Expressionssystems (GibcoBRL) in Sf9 Zellen exprimiert.

Die Testsubstanzen, werden zur Bestimmung ihrer *in vitro* Wirkung an PDE 9A in 100% DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A Präparates hinzugefügt. Die Verdünnung des PDE9A Präparates wird so gewählt, dass während der späteren Inkubation weniger als 70% des Substrates umgesetzt wird (typische Verdünnung: 1: 10000; Verdünnungspuffer: 50 mM Tris/HCl pH 7.5, 8.3 mM MgCl₂, 1.7 mM EDTA, 0.2% BSA). Das Substrat, [8-³H] guanosine 3', 5'-cyclic phosphate (1 µCi/µL; Amersham Pharmacia Biotech., Piscataway, NJ) wird 1:2000 mit Assaypuffer (50 mM Tris/FiCl pH 7.5, 8.3 mM MgCl₂, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDB9A-Inhibitors (z.B. der Inhibitor aus Herstellbeispiel 1, 10 µM Endkonzentration) gestoppt. Direkt im Anschluss werden 25 µL einer Suspension mit 18 mg/mL Yttrium Scintillation Proximity Beads (Amersham Pharmacia Biotech., Piscataway, NJ.) hinzugefügt. Die Mikrotiterplatten werden mit einer Folie versiegelt und für 60 min bei Raumtemperatur stehengelassen. Anschließend werden die Platten für 30 s pro Vertiefung in einem Microbeta Szintillationzähler (Wallac Inc., Atlanta, GA) vermessen. IC₅₀-Werte werden anhand der graphischen Auftragung der Substanzkonzentration gegen die prozentuale Inhibition bestimmt.

Repräsentative Beispiele für die inhibierende Wirkung der erfindungsgemäßen Verbindungen an PDE9A werden anhand der IC₅₀-Werte in Tabelle 1 aufgeführt:

**Tabelle 1**

| Beispiel | IC₅₀-Wert [nM] |
|---|---|
| 2 | 50 |
| 4 | 64 |
| 9 | < 30 |
| 21 | < 30 |

Die *in vitro* Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3', 5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluss an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepasst: Bei PDE1C werden zusätzlich Calmodulin 10⁻⁷ M und CaCl₂ 3mM zum Reaktionsansatz gegeben. PDE2A wird im Test durch Zugabe von cGMP 1 µM stimuliert und mit einer BSA Konzentration von 0,01 % getestet. Für PDE1C und PDE2A wird als Substrat [5',8-³H] adenosine 3', 5'-cyclic phosphate (1 µCi/µL; Amersham Pharmacia Biotech., Piscataway, NJ), für PDE5A [8-³H] guanosine 3', 5'-cyclic phosphate (1 µCi/µL; Amersham Pharmacia Biotech., Piscataway, NJ) eingesetzt.

### Langzeitpotenzierung

Langzeitpotenzierung wird als ein zelluläres Korrelat für Lern- und Gedächinisvorgänge angesehen. Zur Bestimmung, ob PDE9 Inhibition einen Einfluss auf Langzeitpotenzierung hat, kann folgende Methode angewandt werden:

Rattenhippokampi werden in einen Winkel von etwa 70 Grad im Verhältnis zur Schnittklinge plaziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4,9 mM KCl, 1,3 mM MgSO₄* 7H₂O, 2,5 mM CaCl²⁺ Wasser- frei, 1,2 mM KH₂PO₄, 25,6 mM NaHCO₃, 10 mM Glucose, pH 7.4) überführt. Während der Messung befinden sich die Schnitte in einer temperierten Kammer unter einem Flüssigkeitsspiegel von 1-3 mm Höhe. Die Durchflussrate beträgt 2,5 ml/min. Die Vorbegasung erfolgt unter geringen Überdruck (etwa 1 atm) sowie über eine Mikrokanüle in der Vorkammer. Die Schnittkammer ist mit der Vorkammer so verbunden, dass eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatiorischen postsynaptischen Potentiale (fEPSP) 30 % des maximalen exzitatorischen postsynaptischen Potentials (EPSP) betragen. Mit Hilfe einer monopolaren Stimulationselektrode, die aus lackiertem Edelstahl besteht und eines stromkonstanten, biphasischen Reizgenerators (AM-Systems 2100), werden lokal die Schaffer-Kollateralen erregt (Spannung: 1-5 V, Impulsbreite einer Polarität 0,1 ms, Gesamtimpuls 0,2 ms). Mit Hilfe von Glaselektroden (Borosilikatglas mit Filament, 1-5 MOhm, Durchmesser: 1,5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäBen Verbindungen führt zu einer signifikanten Steigerung der LTP.

Die *in vivo*-Wirkung der erfindungsgemäßen Verbindungen kann zum Beispiel wie folgt gezeigt werden:

### Sozialer Wiedererkennungstest:

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt. Nach einer Retentionszeit von 24 Stunden wird der Test wiederholt (Trial 2). Eine verringerte Soziale Interaktionszeit im Vergleich zu Trial 1 zeigt an, dass die adulte Ratte sich an das Jungtier erinnert.

Die adulten Tiere werden entweder in einem festgelegten Zeitabstand (z.B. 1 Stunde) vor Trial 1 oder direkt im Anschluss an Trial 1 entweder mit Vehikel (10 % Ethanol, 20 % Solutol, 70 % physiologische Kochsalzlösung) oder 0,1 mg/kg, 0,3 mg/kg, 1,0 mg/kg bzw. 3,0 mg/kg erfindungsgemäßen Verbindung, gelöst in 10 % Ethanol, 20 % Solutol, 70 % physiologische Kochsalzlösung intraperitoneal injiziert. Vehikel behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel behandelten reduziert. Dies bedeutet, dass die substanzbehandelten Ratten sich an das juvenile Tier erinnert haben und somit die erfindungsgemäßen Verbindungen eine verbessernde Wirkung auf Lernen und Gedächtnis aufweist.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise durch Verstrecken der Wirkstoffe mit Lösungsmitteln und/oder Trägerstoffen, gegebenenfalls unter Verwendung von Emulgiermitteln und/oder Dispergiermitteln hergestellt, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays, oder topisch über die Haut erfolgen.

Im Allgemeinen hat es sich als vorteilhaft erwiesen, Mengen von etwa 0,001 bis 10, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Soweit nicht anders angegeben, beziehen sich alle Mengenangaben auf Gewichtsprozente. Lösungsmittelverhältnisse, Verdunnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

### Abkürzungen:

- DC: Dünnschichtchromatographie
- DCI: direkte chemische Ionisation (bei MS)
- DMSO: Dimethylsulfoxid
- d.Th.: der Theorie (bei Ausbeute)
- ESI: Elektrospray-Ionisation (bei MS)
- Fp.: Schmelzpunkt
- h: Stunde(n)
- HPLC: Hochdruck-, Hochleistungsflüssigchromatographie
- LC-MS: Flüssigchromatographie-gekoppelte Massenspektroskopie
- min: Minuten
- MS: Massenspektroskopie
- NMR: Kernresonanzspektroskopie
- Rₜ: Retentionszeit (bei HPLC)

### LC-MS-Methoden:

### Methode 1

Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50%-ige Ameisensäure, Eluent B: 1 l Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 2

Instrument: Micromass Quattro LCZ, mit HPLC Agilent Serie 1100; Säule: Grom-SIL120 ODS-4 HE, 50 mm x 2.0 mm, 3 µm; Eluent A: 1 l Wasser + 1 ml 50%-ige Ameisensäure, Eluent B: 1 l Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 3

Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 4

Gerätetyp MS: Micromass TOF (LCT); Gerätetyp HPLC: 2-Säulen-Schaltung, Waters 2690; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 3.2 min 10% A; Ofen: 40°C; Fluss: 3.0 ml/min; UV-Detektion: 210 nm.

### Methode 5

Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 500 µl 50%-ige Ameisensäure / 1; Eluent A: Wasser + 500 µl 50%-ige Ameisensäure / l; Gradient: 0.0 min 0% B → 0.2 min 0% B → 2.9 min 70% B → 3.1 min 90% B → 4.5 min 90% B; Ofen: 50°C; Fluss: 0.8 ml/min; UV-Detektion: 210 nm.

### Methode 6

Gerätetyp MS: Micromass ZQ; Gerätetyp HPLC: TSP P4000, TSP AS300, TSP UV3000; Säule: Grom-Sil 120 ODS-4 HE 50 mm x 2 mm, 3.0 µm; Eluent A: Wasser + 250 µl 50%-ige Ameisensäure / l, Eluent B: Acetonitril + 250 µl 50%-ige Ameisensäure / l; Gradient: 0.0 min 0% B → 0.2 min 0% B → 2.9 min 70% B → 3.1 min 90% B → 4.5 min 90% B; Ofen: 50°C; Fluss: 0.8 ml/min; UV-Detektion: 210 nm.

### Ausgangsverbindungen:

### Beispiel 1A

### 5-Amino-1-(2,6-dimethylphenyl)-1H-pyrazol-4-carbonitril

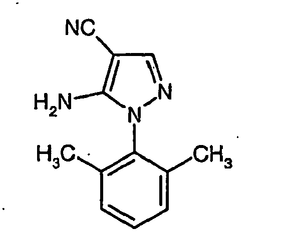

3.0 g (17.3 mmol) 2,6-Dimethylphenylhydrazin-Hydrochlorid werden mit 2.1 g (17.3 mmol) Ethoxymethylenmalonsäuredinitril in 40 ml Ethanol suspendiert und mit 7.3 ml (52.1 mmol) Triethylamin versetzt. Die Reaktionsmischung wird 3 h zum Rückfluss erhitzt, wobei sich eine klare Lösung bildet. Nach Abkühlen auf Raumtemperatur wird diese mit Diethylether versetzt. Das dabei ausfallende Triethylammoniumchlorid wird abfiltriert. Das Lösungsmittel wird im Vakuum entfernt und der Rückstand mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Man erhält 2.3 g (62% d.Th.) des Produktes als gelbe Kristalle.

LC-MS (Methode 6): Rₜ = 2.77 min.

MS (ESI pos): m/z = 213 (M+H)⁺.

### Beispiel 2A

### 5-Amino-1-(2,3-dimethylphenyl)-1H-pyrazol-4-carbonitril

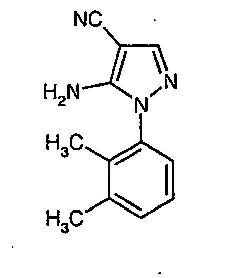

Analog zur Herstellung von Beispiel 1A werden ausgehend von 3 g (17.4 mmol) 2,3-Dimethylphenylhydrazin-Hydrochlorid, 2.1 g (17.4 mmol) Ethoxymethylenmalonsäuredinitril und 7.3 ml (52.1 mmol) Triethylamin 2.08 g (56% d.Th.) des gewünschten Produktes erhalten.

LC-MS (Methode 6): Rₜ = 2.79 min.

MS (ESI pos): m/z = 213 (M+H)⁺.

### Beispiel 3A

### 5-Amino-1-(4-methylphenyl)-1H-pyrazol-4-carbonitril

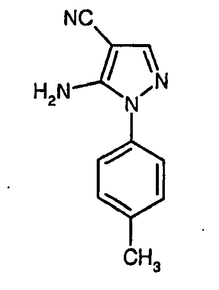

Analog zur Herstellung von Beispiel 1A werden ausgehend von 3 g (18.9 mmol) 4-Methylphenylhydrazin-Hydrochlorid, 2.3 g (18.9 mmol) Ethoxymethylenmalonsäuredinitril und 7.9 ml (56.7 mmol) Triethylamin 2.16 g (57% d.Th.) des gewünschten Produktes erhalten.

LC-MS (Methode 1): Rₜ = 3.0 min.

MS (ESI pos): m/z = 199 (M+H)⁺.

### Beispiel 4A

### 5-Amino-1-(2,6-dichlorphenyl)-1H-pyrazol-4-carbonitril

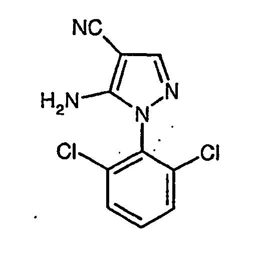

Analog zur Herstellung von Beispiel 1A werden ausgehend von 3 g (14.1 mmol) 2,6-Dichlorphenylhydrazin-Hydrochlorid, 1.7 g (14.1 mmol) Ethoxymethylenmalonsäuredinitril und 5.8 ml (42.2 mmol) Triethylamin nach säulenchromatographischer Reinigung (Laufmittel Dichlormethan/- Methanol 98:2) 2.9 g (83% d.Th.) des gewünschten Produktes erhalten.

LC-MS (Methode 3): Rₜ = 2.8 min.

MS (ESI pos): m/z = 253 (M+H)⁺

¹H-NMR (300 MHz, DMSO-d₆): δ = 6.82 (s, 2H), 7.59 (m, 2H), 7.69 (m. 1H), 7.80 (s, 1H) ppm.

### Beispiel 5A

### 5-Amino-1-(2,5-dichlorphenyl)-1H-pyrazol-4-carbonitril

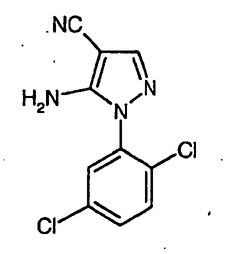

Analog zur Herstellung von Beispiel 1A werden ausgehend von 3 g (16.9 mmol) 2,5-Dichlorphenylhydrazin, 2.0 g (16.9 mmol) Ethoxymethylenmalonsäuredinitril und 7.1 ml (50.8 mmol) Triethylamin 2.2 g (51% d.Th.) des gewünschten Produktes erhalten.

LC-MS (Methode 1): Rₜ = 3.2 min.

MS (ESI pos): m/z = 253 (M+H)⁺.

### Beispiel 6A

### 5-Amino-1-(2-nitrophenyl)-1H-pyrazol-4-carbonitril

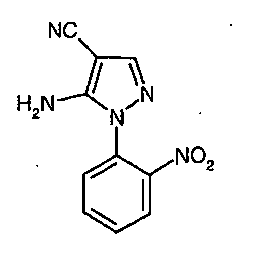

Analog zur Herstellung von Beispiel 1A werden ausgehend von 3 g (15.8 mmol) 2-Nitrophenylhydrazin-Hydrochlorid, 1.93 g (16.9 mmol) Ethoxymethylenmalonsäuredinitril und 6.6 ml (47.6 mmol) Triethylamin 1.9 g (53% d.Th.) des gewünschten Produktes erhalten.

LC-MS (Methode 1): Rₜ = 2.80 min.

MS (ESI pos): m/z = 230 (M+H)⁺

¹H-NMR (300 MHz, DMSO-d₆): δ = 6.87 (s, 2H), 7.72 (m, 1H), 7.77 (s, 1H), 7.78 (m, 1H), 7.88 (m, 1H), 8.16 (dd, 1H) ppm.

### Beispiel 7A

### 5-Amino-1-(3-fluorphenyl)-1H-pyrazol-4-carbonitril

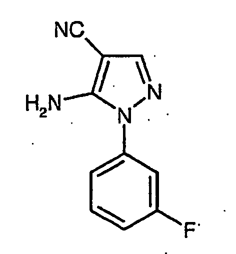

Analog zur Herstellung von Beispiel 1A werden ausgehend von 4 g (24.6 mmol) 3-Fluorphenylhydrazin-Hydrochlorid, 3 g (24.6 mmol) Ethoxymethylenmalonsäuredinitril und 10.3 ml (73.8 mmol) Triethylamin 1.5 g (31% d.Th.) des gewünschten Produktes erhalten.

LC-MS (Methode 1): Rₜ = 2.90 min.

MS (ESI pos): m/z = 203 (M+H)⁺

¹H-NMR (300 MHz, DMSO-d₆): δ = 6.81 (s, 2H), 7.28 (m, 1H), 7.36 (m, 2H), 7.57 (m, 1H), 7.80 (s, 1H) ppm.

### Beispiel 8A

### 5-Amino-1-(3-chlorpyridin-2-yl)-1H-pyrazol-4-carbonitril

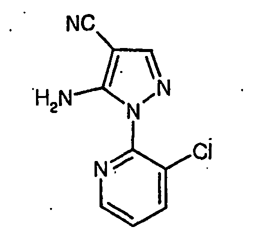

Analog zur Herstellung von Beispiel 1A werden ausgehend von 0.6 g (4.17 mmol) 3-Chlor-2-pyridylhydrazin, 0.51 g (4.17 mmol) Ethoxymethylenmalonsäuredinitril und 1.1 ml (8.3 mmol) Triethylamin 0.4 g (53% d.Th.) des gewünschten Produktes nach säulenchromatographischer Reinigung (Laufmittel Dichlormethan/Methanol 98:2) erhalten.

LC-MS (Methode 6): Rₜ = 2.17 min.

MS (ESI pos): m/z = 220 (M+H)⁺

¹H-NMR (300 MHz, DMSO-d₆): δ = 6.87 (s, 2H), 7.63 (dd, 1H), 7.79 (s, 1H), 8.22 (dd, 1H), 8.54 (dd, 1H) ppm.

### Beispiel 9A

### 5-Amino-1-(2-methylphenyl)-1H-pyrazol-4-carbonitril

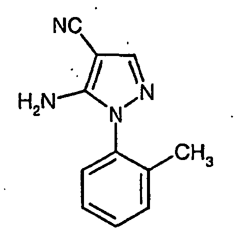

10.2 g (64.4 mmol) 2-Methylphenylhydrazin-Hydrochlorid werden mit 7.8 g (64.4 mmol) Ethoxymethylenmalonsäuredinitril in 100 ml Methanol suspendiert und mit 26.9 ml (193.3 mmol) Triethylamin versetzt. Die Reaktionsmischung wird über Nacht zum Rückfluss erhitzt, wobei sich eine klare Lösung bildet. Das Lösungsmittel wird anschließend unter reduziertem Druck abdestilliert und das Rohprodukt säulenchromatographisch aufgereinigt (Kieselgel, Laufmittel Dichlormethan). Es werden 10.8 g (85% d.Th.) des gewünschten Produktes erhalten.

LC-MS (Methode 1): Rₜ = 3.10 min.

MS (ESI pos): m/z = 199 (M+H)⁺.

### Beispiel 10A

### 5-Amino-1-(2-ethylphenyl)-1H-pyrazol-4-carbonitril

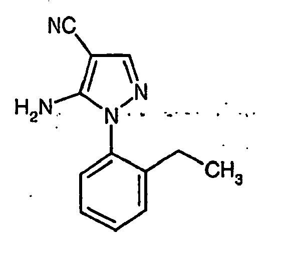

Eine Lösung von 3.0 g (17.0 mmol) 2-Ethylphenylhydrazin-Hydrochlorid und 2.12 g (17.0 mmol) Ethoxymethylenmalonsäuredinitril in 36 ml Ethanol wird mit 7.1 ml (51.1 mmol) Triethylamin versetzt und auf 60°C erhitzt, bis die Umsetzung laut DC-Kontrolle vollständig ist (ca. 30 min). Zur Aufarbeitung wird das Lösungsmittel abgezogen, der Rückstand in Dichlormethan aufgenommen, mit gesättigter Natriumhydrogencarbonat-Lösung gewaschen und über Natriumsulfat getrocknet. Nach dem Einengen erhält man ein Rohprodukt, das durch Säulenchromatographie an Kieselgel (Laufmittel Dichlormethan mit 0-10% Methanol) gereinigt wird. Es werden 3.05 g (83.5% d.Th.) des gewünschten Produktes erhalten.

Fp.: 130°C

MS (ESI pos): m/z = 213 (M+H)⁺

¹H-NMR (300 MHz, DMSO-d₆): δ = 1.0 (t, 3H), 2.35 (q, 2H), 6.4 (s, 2H), 7.2-7.5 (m, 4H), 7.7 (s, 1H) ppm.

### Beispiel 11A

### 5-Amino-1-(2-trifluormethylphenyl)-1H-pyrazol-4-carbonitril

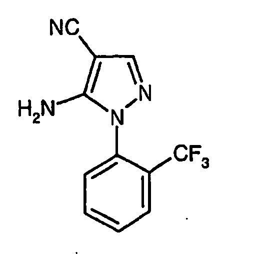

Analog zur Herstellung von Beispiel 10A werden ausgehend von 4.8 g (25.9 mmol) 2-Trifluormethylphenylhydrazin-Hydrochlorid, 3.16 g (25.9 mmol) Ethoxymethylen-malonsäuredinitril und 7.2 ml (51.7 mmol) Triethylamin 5.02 g (76.9% d.Th.) des gewünschten Produktes erhalten.

Fp.: 190°C

MS (ESI pos): m/z = 253 (M+H)⁺

¹H-NMR (300 MHz, DMSO-d₆): δ = 6.6 (s, 2H), 7.5 (d, 1H), 7.7-8.0 (m, 4H) ppm.

### Beispiel 12A

### 5-Amino-1-(2-fluorphenyl)-1H-pyrazol-4-carbonitril

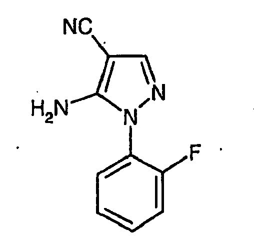

Analog zur Herstellung von Beispiel 10A werden ausgehend von 5.0 g (30.8 mmol) 2-Fluorphenylhydrazin-Hydrochlorid, 3.27 g (26.7 mmol) Ethoxymethylenmalonsäuredinitril und 11.3 ml (81.3 mmol) Triethylamin 5.13 g (88% Reinheit, 84% d.Th.) des gewünschten Produktes erhalten.

MS (ESI pos): m/z = 203 (M+H)⁺

¹H-NMR (300 MHz, DMSO-d₆): δ = 6.7 (s, 2H), 7.3-7.6 (m, 4H), 7.8 (s, 1H) ppm.

### Beispiel 13A

### 5-Amino-1-(2-chlorphenyl)-1H-pyrazol-4-carbonitril

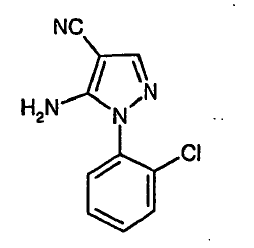

Analog zur Herstellung von Beispiel 10A werden ausgehend von 5.0 g (27.1 mmol) 2-Chlorphenylhydrazin-Hydrochlorid, 3.31 g (27.1 mmol) Ethoxymethylenmalonsäuredinitril und 11.3 ml (81.3 mmol) Triethylamin 4.64 g (78% d.Th.) des gewünschten Produktes erhalten:

Fp.: 135°C

MS (ESI pos): m/z = 219 (M+H)⁺

¹H-NMR (300 MHz, DMSO-d₆): δ = 6.6 (s; 2H), 7.45-7.75 (m, 4H), 7.8 (s, 1H) ppm

### Beispiel 14A

### 5-Amino-1-(2-pyridinyl)-1H-pyrazol-4-carbonitril

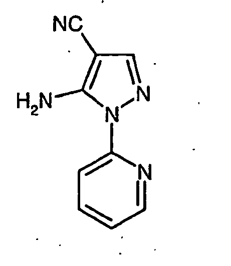

Analog zur Herstellung von Beispiel 10A werden ausgehend von 3.0 g (26.7 mmol, 97% Reinheit) 2-Hydrazinopyridin, 3.26 g (26.7 mmol) Ethoxymethylenmalonsäuredinitril und 7.4 ml (53.3 mmol) Triethylamin 2.3 g (46.6% d.Th.) des gewünschten Produktes erhalten.

Fp.: 193°C

MS (ESI pos): m/z = 186 (M+H)⁺

¹H-NMR (300 MHz, DMSO-d₆): δ = 7.35 (m, 1H), 7.8-8.12 (m, 3H), 8.15 (s, 2H), 8.5 (m, 1H) ppm.

### Beispiel 15A

### 5-Amino-1-(2-methoxyphenyl)-1H-pyrazol-4-carbonitril

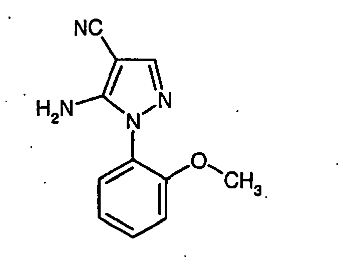

Analog zur Herstellung von Beispiel 10A werden ausgehend von 4.1 g (18 mmol) 2-Methoxyphenylhydrazin-Hydrochlorid, 2.19 g (18 mmol) Ethoxymethylenmalonsäuredinitril und 10 ml (71.9 mmol) Triethylamin 3.5 g (88% d.Th.) des gewünschten Produktes erhalten.

Fp.: 129°C

MS (ESI pos): m/z = 215 (M+H)⁺

¹H-NMR (300 MHz, DMSO-d₆): δ = 3.8 (s, 3H), 6.3 (s, 2H), 7.05 (t, 1H), 7.2 (d, 1H), 7.25 (d, 1H), 7.5 (t, 1H), 7.7 (s, 1H) ppm.

### Beispiel 16A

### 5-Amino-1-(2,6-dimethylphenyl)-1H-pyrazol-4-carbonsäureamid

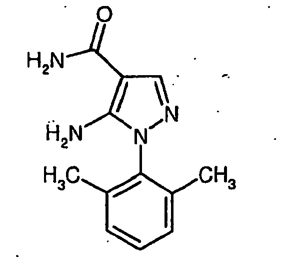

2 g (9.4 mmol) 5-Amino-1-(2,6-dimethylphenyl)-1H-pyrazol-4-carbonitril (Beispiel 1A) werden in 25 ml Ethanol gelöst und mit einer Mischung aus 20 ml 30%-igem Wasserstoffperoxid und 40 ml 25%-igem Ammoniak versetzt. Man rührt über Nacht bei Raumtemperatur und engt anschließend am Rotationsverdampfer die Lösung bis auf ca. 15 ml ein. Die dabei entstehende ölige Emulsion wird in Dichlormethan aufgenommen. Man wäscht mehrfach mit Wasser und gesättigter Natriumthiosulfat-Lösung. Nach Trocknen über Magnesiumsulfat wird das Lösungsmittel im Vakuum entfernt. Der Rückstand wird mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Es werden 0.88 g (40% d.Th.) des Produktes als farbloser Feststoff erhalten.

LC-MS (Methode 1): Rₜ = 2.6 min.

MS (ESI pos): m/z = 231 (M+H)⁺.

### Beispiel 17A

### 5-Amino-1-(2,3-dimethylphenyl)-1H-pyrazol-4-carbonsäureamid

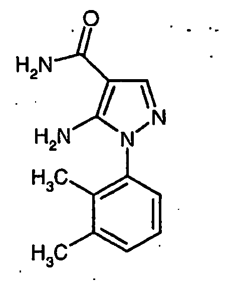

Analog zur Herstellung von Beispiel 16A werden aus 1.5 g (7.1 mmol) 5-Amino-1-(2,3-dimethylphenyl)-1H-pyrazol-4-carbonitril (Beispiel 2A) in einer Mischung aus 25 ml Ethanol, 10 ml 30%-igem Wasserstoffperoxid und 40 ml 25%-igem Ammoniak 1.29 g (70% d.Th.) des gewünschten Produktes erhalten.

LC-MS (Methode 1): Rₜ = 2.7 min.

MS (ESI pos): m/z = 231 (M+H)⁺.

### Beisipiel 18A

### 5-Amino-1-(4-methylphenyl)-1H-pyrazol-4-carbonsäureamid

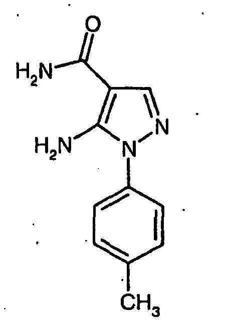

Analog zur Herstellung von Beispiel 16A werden aus 2 g (10.1 mmol) 5-Amino-1-(4-methylphenyl)-1H-pyrazol-4-carbonitril (Beispiel 3A) in einer Mischung aus 25 ml Ethanol, 20 ml 30%-igem Wasserstoffperoxid und 40 ml 25%-igem Ammoniak 1.02 g (47% d.Th.) des gewünschten Produktes erhalten.

LC-MS (Methode 1): Rₜ = 2.7 min.

MS (ESI pos): m/z = 217 (M+H)⁺.

### Beispiel 19A

### 5-Amino-1-(2,6-dichlorphenyl)-1H-pyrazol-4-carbonsäureamid

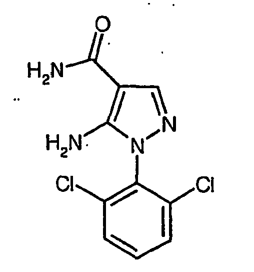

Analog zur Herstellung von Beispiel 16A werden aus 2 g (7.9 mmol) 5-Amino-1-(2,6-dichlorphenyl)-1H-pyrazol-4-carbonitril (Beispiel 4A) in einer Mischung aus 25 ml Ethanol, 10 ml 30%-igem Wasserstoffperoxid und 40 ml 25%-igem Ammoniak 1.6 g (74% d.Th.) des gewünschten Produktes durch Kristallisation aus der Reaktionslösung erhalten.

LC-MS (Methode 1): Rₜ = 2.5 min.

MS (ESI pos): m/z = 271 (M+H)⁺.

### Beispiel 20A

### 5-Amino-1-(2,5-dichlorphenyl)-1H-pyrazol-4-carbonsäureamid

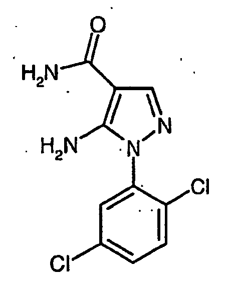

Analog zur Herstellung von Beispiel 16A werden aus 2 g (7.9 mmol) 5-Amino-1-(2,5-dichlorphenyl)-1H-pyrazol-4-carbonitril (Beispiel 5A) in einer Mischung aus 25 ml Ethanol, 18 ml 30%-igem Wasserstoffperoxid und 40 ml 25%-igem Ammoniak 2.02 g (94% d.Th.) des gewünschten Produktes durch Kristallisation aus der Reaktionslösung erhalten.

LC-MS (Methode 1): Rₜ = 2.80 min.

MS (ESI pos): m/z = 271 (M+H)⁺.

### Beispiel 21A

### 5-Amino-1-(2-nitrophenyl)-1H-pyrazol-4-carbonsäureamid

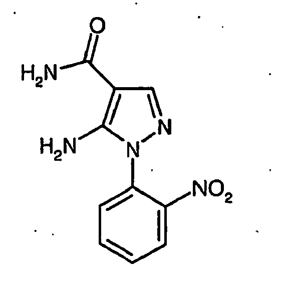

Analog zur Herstellung von Beispiel 16A werden aus 1.5 g (6.5 mmol) 5-Amino-1-(2-nitrophenyl)-1H-pyrazol-4-carbonitril (Beispiel 6A) in einer Mischung aus 25 ml Ethanol, 16 ml 30%-igem Wasserstoffperoxid und 40 ml 25%-igem Ammoniak 1.4 g (86% d.Th.) des gewünschten Produktes durch Kristallisation aus der Reaktionslösung erhalten.

LC-MS (Methode 1): Rₜ = 2.3 min.

MS (ESI pos): m/z = 248 (M+H)⁺_{.}

### Beispiel 22A

### 5-Amino-1-(2-aminophenyl)-1H-pyrazol-4-carbonsäureamid

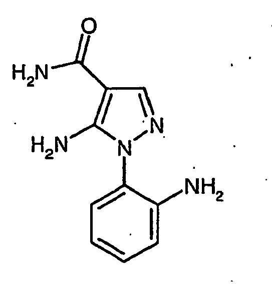

1.28 g (5.27 mmol) 5-Amino-1-(2-mtrophenyl)-1H-pyrazol-4-carbonsäureamid (Beispiel 21A) werden in 30 ml Essigsäureethylester vorgelegt und mit 5.8 g (25.8 mmol) Zinn(II)chlorid-Dihydrat 16 h lang bei 70°C gerührt. Nach Abkühlen auf Raumtemperatur wird die Lösung mit gesättigter Natriumhydrogencarbonat-Lösung auf pH 9-10 gebracht. Die dabei ausfallenden Zinnsalze werden über Kieselgur abfiltriert. Das Filtrat wird mit Essigsäureethylester extrahiert. Die vereinigten organischen Phasen werden mit gesättigter Natriumchlorid-Lösung gewaschen. Nach Trocknen über Natriumsulfat wird das Lösungsmittel im Vakuum entfernt. Man erhält 0.82 g (72% d.Th.) des gewünschten Produktes.

LC-MS (Methode 2): Rₜ = 3.0 min.

MS (ESI pos): m/z = 218 (M+H)⁺

¹H-NMR (300 MHz, DMSO-d₆): δ = 5.04 (s, 2H), 6.00 (s, 2H), 6.66 (m, 1H), 6.89 (m, 1H), 7.03 (m, 2H), 7.92 (s, 1H) ppm.

### Beispiel 23A

### 5-Amino-1-(3-fluorphenyl)-1H-pyrazol-4-carbonsäureamid

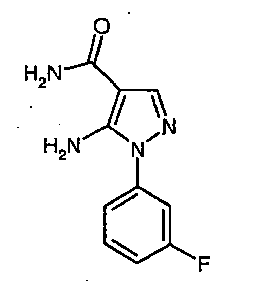

Analog zur Herstellung von Beispiel 16A werden aus 1.3 g (6.4 mmol) 5-Amino-1-(3-fluorphenyl)-1H-pyrazol-4-carbonitril (Beispiel 7A) in einer Mischung aus 25 ml Ethanol, 10 ml 30%-igem Wasserstoffperoxid und 40 ml 25%-igem Ammoniak 1.1 g (75% d.Th.) des gewünschten Produktes durch Kristallisation aus der Reaktionslösung erhalten.

LC-MS (Methode 1): Rₜ = 2.60 min.

MS (ESI pos): m/z = 221 (M+H)⁺.

### Beispiel 24A

### 5-Amino-1-(3-chlorpyridin-2-yl)-1H-pyrazol-4-carbonsäureamid

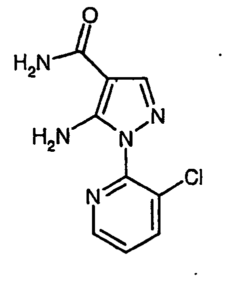

Analog zur Herstellung von Beispiel 16A werden aus 0.4 g (1.8 mmol) 5-Amino-1-(3-chlorpyridin-2-yl)-1H-pyrazol-4-carbonitril (Beispiel 8A) in einer Mischung aus 7 ml Ethanol, 5 ml 30%-igem Wasserstoffperoxid und 7 ml 25%-igem Ammoniak 0.29 g (66% d.Th.) des gewünschten Produktes erhalten.

LC-MS (Methode 2): Rₜ = 3.00 min.

MS (ESI pos): m/z = 238 (M+H)⁺

¹H-NMR (300 MHz, DMSO-d₆): δ = 6.42 (s, 2H), 7.58 (dd, 1H), 7.88 (s, 1H), 8.20 (dd, 1H), 8.53 (dd, 1H) ppm.

### Beispiel 25A

### 5-Amino-1-(2-methylphenyl)-1H-pyrazol-4-carbonsäureamid

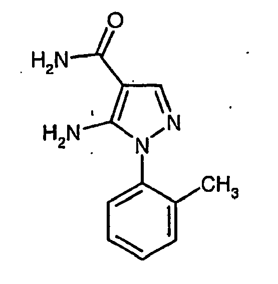

40.0 g (201.8 mmol) 5-Amino-1-(2-methylphenyl)-1H-pyrazol-4-carbonitril (Beispiel 9A) werden unter Eiskühlung vorsichtig mit 300 ml 96%-iger Schwefelsäure versetzt. Anschließend wird auf 40°C erhitzt und 2 h lang bei dieser Temperatur gerührt. Nach dem Abkühlen wird auf 2 l Eiswasser gegossen und vorsichtig mit 50%-iger Natriumhydroxid-Lösung neutralisiert. Nach dreimaliger Extraktion mit Essigsäureethylester (jeweils 2 1) werden die vereinigten organischen Phasen mit gesättigter Natriumchlorid-Lösung gewaschen, über Natriumsulfat getrocknet und das Lösungsmittel unter reduziertem Druck abdestilliert. Es werden 36.0 g (82% d.Th.) Produkt (Reinheit >90%) erhalten, welches ohne weitere Aufreinigung in Folgereaktionen eingesetzt wird.

LC-MS (Methode 6): Rₜ = 2.14 min.

MS (ESI pos): m/z = 217 (M+H)⁺.

### Beispiel 26A

### 5-Amino-1-(2-ethylphenyl)-1H-pyrazol-4-carbonsäureamid

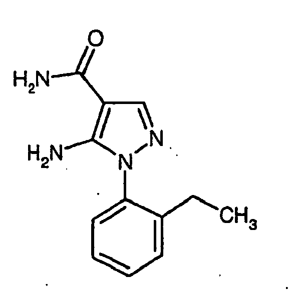

Analog zur Herstellung von Beispiel 16A werden aus 2.75 g (12.8 mmol) 5-Amino-1-(2-ethylphenyl)-1H-pyrazol-4-carbonitril (Beispiel 10A) in einer Mischung aus 106 ml Ethanol, 27 ml 30%-igem Wasserstoffperoxid und 133 ml 25%-igem Ammoniak 2.58 g (87% d.Th.) des gewünschten Produktes nach Kieselgelchromatographie (Laufmittel Dichlormethan mit 0-10% Methanol) erhalten.

Fp.: 147°C

MS (ESI pos): m/z = 231 (M+H)⁺

¹H-NMR (300 MHz, DMSO-d₆): δ = 1.0 (t, 3H), 2.4 (q, 2H), 5.95 (s, 2H), 6.3 (breites d, 2H), 7.2-7.5 (m, 4H), 7.8 (s, 1H) ppm.

### Beispiel 27A

### 5-Amino-1-(2-trifluormethylphenyl)-1H-pyrazol-4-carbonsäureamid

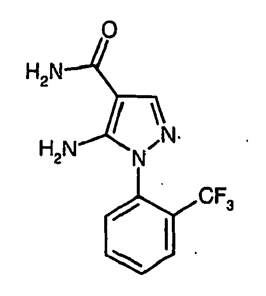

Analog zur Herstellung von Beispiel 16A werden aus 5.0 g (19.8 mmol) 5-Amino-1-(2-trifluormethylphenyl)-1H-pyrazol-4-carbonitril (Beispiel 11A) in einer Mischung aus 195 ml Ethanol, 49 ml 30%-igem Wasserstoffperoxid und 244 ml 25%-igem Ammoniak 4.01 g (87% d.Th.) des gewünschten Produktes nach Kieselgelchromatographie (Laufmittel Dichlormethan mit 0-10% Methanol) erhalten.

Fp.: 186°C

MS (ESI pos): m/z = 271 (M+H)⁺

¹H-NMR (300 MHz, DMSO-d₆): δ = 6.1 (s, 2H), 7.0 (breites d, 2H), 7.45-8.0 (m, 5H) ppm.

### Beispiel 28A

### 5-Amino-1-(2-fluorphenyl)-1H-pyrazol-4-carbonsäureamid

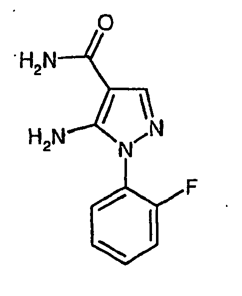

Analog zur Herstellung von Beispiel 16A werden aus 5.0 g (21.9 mmol, 89% Reinheit) 5-Amino-1-(2-fluorphenyl)-1H-pyrazol-4-carbonitril (Beispiel 12A) in einer Mischung aus 173 ml Ethanol, 43 ml 30%-igem Wasserstoffperoxid und 216 ml 25%-igem Ammoniak 3.89 g (81% d.Th.) des gewünschten Produktes nach Kieselgelchromatographie (Laufmittel Dichlormethan mit 0-10% Methanol) erhalten.

Fp.: 181°C

MS (ESI pos): m/z = 221 (M+H)⁺

¹H-NMR (300 MHz, DMSO-d₆): δ = 6.2 (s, 2H), 7.0 (breites d, 2H), 7.3-7.6 (m, 4H), 7.9 (s, 1H) ppm.

### Beispiel 29A

### 5-Amino-1-(2-chlorphenyl)-1H-pyrazol-4-carbonsäureamid

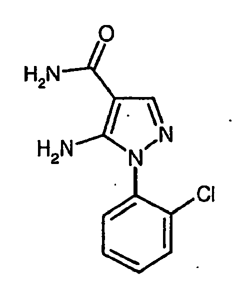

Analog zur Herstellung von Beispiel 16A werden aus 4.6 g (21.0 mmol) 5-Amino-1-(2-chlorphenyl)-1H-pyrazol-4-carbonitril (Beispiel 13A) in einer Mischung aus 159 ml Ethanol, 39 ml 30%-igem Wasserstoffperoxid und 198 ml 25%-igem Ammoniak 3.93 g (79% d.Th.) des gewünschten Produktes nach Kieselgelchromatographie (Laufmittel Dichlormethan mit 0-10% Methanol) erhalten.

Fp.: 166°C

MS (ESI pos): m/z = 237 (M+H)⁺

¹H-NMR (300 MHz, DMSO-d₆): δ = 6.1 (s, 2H), 7.0 (breites d, 2H), 7.4-7.7 (m, 4H), 7.85 (s, 1H) ppm.

### Beispiel 30A

### 5-Amino-1-(2-pyridinyl)-1H-pyrazol-4-carbonsäureamid

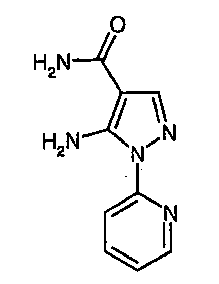

Analog zur Herstellung von Beispiel 16A werden aus 2.3 g (12.4 mmol) 5-Amino-1-(2-pyridinyl)-1H-pyrazol-4-carbonitril (Beispiel 14A) in einer Mischung aus 90 ml Ethanol, 23 ml 30%-igem Wasserstoffperoxid und 113 ml 25%-igem Ammoniak 2.28 g (90% d.Th.) des gewünschten Produktes nach Kieselgelchromatographie (Laufmittel Dichlormethan mit 0-10% Methanol) erhalten.

Fp.: 218°C

MS (DCI): m/z = 204 (M+H)⁺

¹H-NMR (300 MHz, DMSO-d₆): δ = 7.1 (breites d, 2H), 7.3 (dd, 1H), 7.5 (s, 2H), 7.85 (d, 1H), 7.95 (s, 1H), 8.0 (dd, 1H), 8.45 (d, 1H) ppm.

### Beispiel 31A

### 5-Amino-1-(2-methoxyphenyl)-1H-pyrazol-4-carbonsäureamid

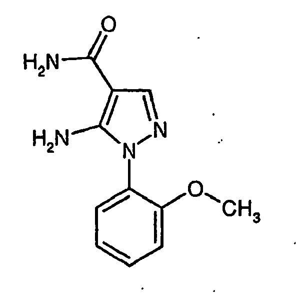

Analog zur Herstellung von Beispiel 16A werden aus 3.5 g (16.0 mmol, 98% Reinheit) 5-Amino-1-(2-methoxyphenyl)-1H-pyrazol-4-carbonitril (Beispiel 15A) in einer Mischung aus 172 ml Ethanol, 34 ml 30%-igem Wasserstoffperoxid und 137 ml 25%-igem Ammoniak 2.61 g (70% d.Th.) des gewünschten Produktes nach Kieselgelchromatographie (Laufmittel Dichlormethan mit 0-10% Methanol) erhalten.

Fp.: 191°C

MS (ESI pos): m/z = 233 (M+H)⁺

¹H-NMR (300 MHz, DMSO-d₆): δ = 3.8 (s, 3H), 5.9 (s, 2H), 7.0 (breites s, 2H), 7.05-7.55 (m, 4H), 7.8 (s, 1H) ppm.

### Ausführungsbeispiele:

### Beispiel 1

### 6-(3-Chlorbenzyl)-1-(2,6-dimethylphenyl)-1,5-dihydro-pyrazolo[3,4-d]pyrimidin-4-on

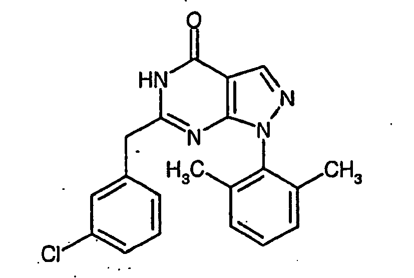

0.1 g (0.43 mmol) 5-Amino-1-(2,6-dimethylphenyl)-1H-pyrazol-4-carbonsäureamid (Beispiel 16A) werden unter Argon in 6 ml absolutem Ethanol gelöst und mit 0.24 g (1.3 mmol) 3-Chlorphenylessigsäuremethylester und 0.17 g (4.34 mmol) 60%-igem Natriumhydrid (Suspension in Mineralöl) versetzt. Man erhitzt die Reaktionsmischung über Nacht zum Rückfluss. Nach Abkühlen auf Raumtemperatur wird mit konzentrierter Salzsäure angesäuert. Das dabei ausfallende Natriumchlorid wird abfiltriert. Das Filtrat wird im Vakuum eingeengt und der verbleibende Rückstand mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B). Es werden 59 mg (37% d.Th.) des Produktes als farbloser Feststoff erhalten.

LC-MS (Methode 2): Rₜ = 4.20 min.

MS (ESI pos): m/z = 365 (M+H)⁺

¹H-NMR (300 MHz, DMSO-d₆): δ = 1.87 (s, 6H), 3.92 (s, 2H), 7.29 (m, 7H), 8.28 (s, 1H), 12.43 (s, 1H) ppm.

### Beispiel 2

### 6-(3-Chlorbenzyl)-1-(2,3-dimethylphenyl)-1,5-dihydro-pyrazolo[3,4-d]pyrimidin-4-on

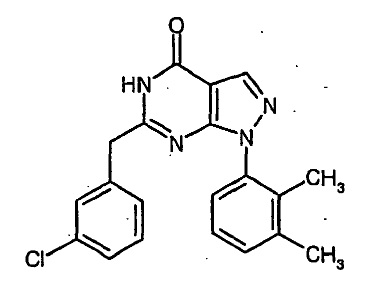

0.1 g (0.43 mmol) 5-Amino-1-(2,3-dimethylphenyl)-1H-pyrazol-4-carbonsäureamid (Beispiel 17A) werden unter Argon in 6 ml absolutem Ethanol gelöst und mit 0.24 g (1.3 mmol) 3-Chlorphenylessigsäuremethylester und 0.17 g (4.34 mmol) 60%-igem Natriumhydrid (Suspension in Mineralöl) versetzt. Man erhitzt die Reaktionsmischung über Nacht zum Rückfluss. Nach Abkühlen auf Raumtemperatur wird mit konzentrierter Salzsäure angesäuert. Die dabei ausfallende Mischung aus Natriumchlorid und dem Produkt wird abfiltriert und mehrfach mit Wasser und Diethylether gewaschen. Nach Trocknen im Hochvakuum werden 97 mg (61% d.Th.) des Produktes als farbloser Feststoff erhalten.

LC-MS (Methode 3): Rₜ = 3.85 min.

MS (ESI pos): m/z = 365 (M+H)⁺

¹H-NMR (300 MHz, DMSO-d₆): δ = 1.82 (s, 3H), 2.32 (s, 3H), 3.92 (s, 2H), 7.31 (m, 7H), 8.23 (s, 1H), 12.40 (s, 1H) ppm.

### Beispiel 3

### 6-(3-Chlorbenzyl)-1-(4-methylphenyl)-1,5-dihydro-pyrazolo[3,4-d]pyrimidin-4-on

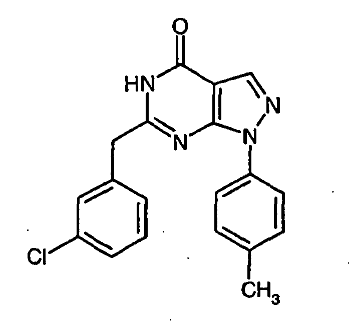

Analog zur Herstellung von Beispiel 2 werden ausgehend von 0.88 g (0.41 mmol) 5-Amino-1-(4-methylphenyl)-1H-pyrazol-4-carbonsäureamid (Beispiel 18A), 0.22 g (1.2 mmol) 3-Chlorphenylessigsäuremethylester und 0.16 g (4.09 mmol) 60%-igem Natriumhydrid 99 mg (69% d.Th.) des gewünschten Produktes als farbloser Feststoff erhalten.

LC-MS (Methode 3): Rₜ = 4.03 min.

MS (ESI pos): m/z = 351 (M+H)⁺

¹H-NMR (300 MHz, DMSO-d₆): δ = 2.35 (s, 3H), 4.04 (s, 2H), 7.35 (m, 5H), 7.50 (s, 1H), 7.89 (d, 2H), 8.23 (s, 1H), 12.49 (s, 1H) ppm.

### Beispiel 4

### 6-(3-Chlorbenzyl)-1-(2,6-dichlorphenyl)-1,5-dihydro-pyrazolo[3,4-d]pyrimidin-4-on

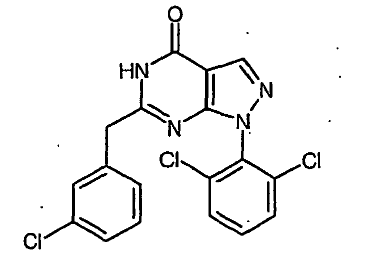

Analog zur Herstellung von Beispiel 1 werden ausgehend von 0.1 g (0.37 mmol) 5-Amino-1-(2,6-dichlorphenyl)-1H-pyrazol-4-carbonsäureamid (Beispiel 19A), 0.2 g (1.1 mmol) 3-Chlorphenylessigsäuremethylester und 0.14 g (3.6 mmol) 60%-igem Natriumhydrid 104 mg (69% d.Th.) des gewünschten Produktes als farbloser Feststoff erhalten.

LC-MS (Methode 3): Rₜ = 3.77 min.

MS (ESI pos): m/z = 405 (M+H)⁺

¹H-NMR (300 MHz, DMSO-d₆): δ = 3.94 (s, 2H), 7.30 (m, 4H), 7.69 (m, 2H), 7.70 (s, 1H), 8.39 (s, 1H), 12.57 (s, 1H) ppm

### Beispiel 5

### 6-(3-Chlorbenzyl)-1-(2,5-dichlorphenyl)-1,5-dihydro-pyrazolo[3,4-d]pyrimidin-4-on

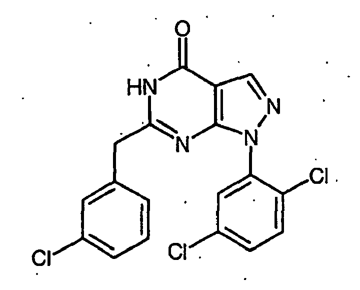

Analog zur Herstellung von Beispiel 1 werden ausgehend von 0.1 g (0.37 mmol) 5-Amino-1-(2,5-dichlorphenyl)-1H-pyrazol-4-carbonsäureamid (Beispiel 20A), 0.2 g (1.1 mmol) 3-Chlorphenylessigsäuremethylester und 0.14 g (3.6 mmol) 60%-igem Natriumhydrid 35 mg (23% d.Th.) des gewünschten Produktes als farbloser Feststoff erhalten.

LC-MS (Methode 2): Rₜ = 4.20 min.

MS (ESI pos): m/z = 405 (M+H)⁺.

### Beispiel 6

### 1-(2-Aminophenyl)-6-(3-chlorbenzyl)-1,5-dihydro-pyrazolo[3,4-d]pyrimidin-4-on

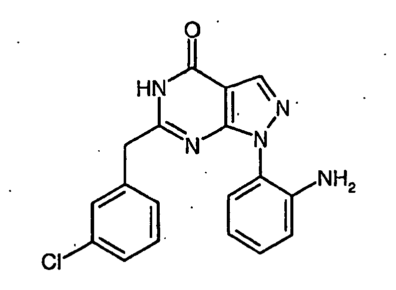

Analog zur Herstellung von Beispiel 1 werden ausgehend von 0.1 g (0.46 mmol) 5-Amino-1-(2-aminophenyl)-1H-pyrazol-4-carbonsäureamid (Beispiel 22A), 0.25 g (1.4 mmol) 3-Chlorphenylessigsäuremethylester und 0.18 g (4.6 mmol) 60%-igem Natriumhydrid 103 mg (63% d.Th.) des gewünschten Produktes als farbloser Feststoff erhalten.

LC-MS (Methode 6): Rₜ = 3.32 min.

MS (ESI pos): m/z = 352 (M+H)⁺

¹H-NMR (300 MHz, DMSO-d₆): δ = 3.96 (s, 2H), 4.46 (s, 2H), 6.81 (t, 1H), 7.03 (d, 1H), 7.31 (m, 5H), 7.44 (s, 1H), 8.28 (s, 1H), 12.47 (s, 1H) ppm.

### Beispiel 7

### 6-(3-Chlorbenzyl)-1-(3-fluorphenyl)-1,5-dihydro-pyrazolo[3,4-d]pyrimidin-4-on

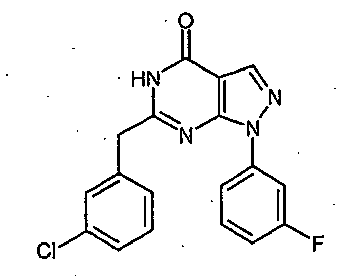

Analog zur Herstellung von Beispiel 2 werden ausgehend von 0.1 g (0.45 mmol) 5-Amino-1-(3-fluorphenyl)-1H-pyrazol-4-carbonsäureamid (Beispiel 23A), 0.25 g (1.36 mmol) 3-Chlorphenylessigsäuremethylester und 0.18 g (4.5 mmol) 60%-igem Natriumhydrid 125 mg (77% d.Th.) des gewünschten Produktes als farbloser Feststoff erhalten.

LC-MS (Methode 3): Rₜ = 3.98 min.

MS (ESI pos): m/z = 355 (M+H)⁺

¹H-NMR (300 MHz, DMSO-d₆): δ = 4.08 (s, 2H), 7.19 (m, 1H), 7.36 (m, 3H), 7.55 (m, 2H), 7.93 (m, 2H), 8.3 (s, 1H), 12.61 (s, 1H) ppm.

### Beispiel 8

### 6-(3-Chlorbenzyl)-1-(3-chlorpyridin-2-yl)-1,5-dihydro-pyrazolo[3,4-d]pyrimidin-4-on

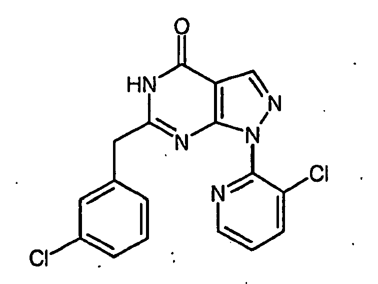

Analog zur Herstellung von Beispiel 2 werden ausgehend von 0.1 g (0.42 mmol) 5-Amino-1-(3-chlorpyridin-2-yl)-1H-pyrazol-4-carbonsäureamid (Beispiel 24A), 0.23 g (1.26 mmol) 3-Chlorphenylessigsäuremethylester und 0.17 g (4.2 mmol) 60%-igem Natriumhydrid 85 mg (54% d.Th.) des gewünschten Produktes als farbloser Feststoff erhalten.

LC-MS (Methode 3): Rₜ = 3.10 min.

MS (ESI pos): m/z = 372 (M+H)⁺

¹H-NMR (300 MHz, DMSO-d₆): δ = 4.01 (s, 2H), 7.34 (m, 4H), 7.49 (s, 1H), 7.69 (d, 1H), 7.91 (t, 1H), 8.27 (s, 1H), 12.57 (s, 1H) ppm.

### Beispiel 9

### 6-(2-Brombenzyl)-1-(2-methylphenyl)-1,5-dihydro-pyrazolo[3,4-d]pyrimidin-4-on

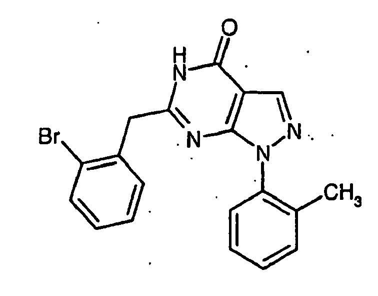

2.0 g (9.25 mmol) 5-Ammo-1-(2-methylphenyl)-1H-pyrazol-4-carbonsäureamid (Beispiel 25A) und 9.5 g (41.62 mmol) (2-Bromphenyl)essigsäuremethylester werden unter Argon in 30 ml absolutem Ethanol gelöst und mit 3.15 g (46.6 mmol) Natriumethylat versetzt. Man erhitzt die Reaktionsmischung über Nacht zum Rückfluss. Nach Abkühlen auf .Raumtemperatur wird mit 50 ml Wasser hydrolysiert und anschließend mit Essigsäureethylester (2 x 50 ml) extrahiert. Die vereinigten organischen Phasen werden über Natriumsulfat getrocknet und das Lösungsmittel unter reduziertem Druck abdestilliert. Das Rohprodukt wird anschließend mittels Säulenchromatographie aufgereinigt (Kieselgel; Laufmittel Cyclohexan/Essigsäureethylester, Gradient 10:1 → 1:1). Es werden 3.01 g (82% d.Th.) des Produkts erhalten.

LC-MS (Methode 5): Rₜ = 3.27 min.

MS (ESI pos): m/z = 395 (M+H)⁺

¹H-NMR (300 MHz, DMSO-d₆): δ = 1.96 (s, 3H), 4.09 (s, 2H), 7.14-7.40 (7H), 7.59 (dd, 1H), 8.26 (s, 1H), 12.55 (s, 1H) ppm.

### Beispiel 10

### 6-(3-Brombenzyl)-1-(2-methylphenyl)-1,5-dihydro-pyrazolo[3,4-d]pyrimidin-4-on

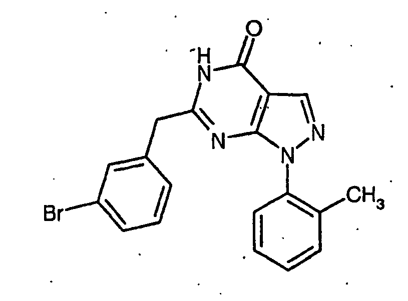

813 mg (3.78 mmol) 5-Amino-1-(2-methylphenyl)-1H-pyrazol-4-carbonsäureamid (Beispiel 25A) und 3.90 g (17.03 mmol) (3-Bromphenyl)essigsäuremethylester werden unter Argon in 15 ml absolutem Ethanol gelöst und mit 1.29 g (18.9 mmol) Natriumethylat versetzt. Man erhitzt die Reaktionsmischung über Nacht zum Rückfluss. Nach Abkühlen auf Raumtemperatur wird mit 25 ml Wasser hydrolysiert und anschließend mit Essigsäureethylester (2 x 25 ml) extrahiert. Die vereinigten organischen Phasen werden über Natriumsulfat getrocknet und das Lösungsmittel unter reduziertem Druck abdestilliert. Das Rohprodukt wird anschließend mittels Säulenchromatographie aufgereinigt (Kieselgel; Laufmittel Cycloexan/Essigsäureethylester, Gradient 10:1 → 1:1). Es werden 1.33 g (89% d.Th.) des Produkts erhalten.

LC-MS (Methode 5): Rₜ = 3.34 min.

MS (ESI pos): m/z = 395 (M+H)⁺

¹H-NMR (300 MHz, DMSO-d₆): δ = 2.03 (s, 3H), 3.92 (s, 2H), 7.24-7.51 (7H), 7.56 (m, 1H), 8.22 (s, 1H), 12.45 (s, 1H) ppm.

Die in der folgenden Tabelle 1 aufgeführten Ausführungsbeispiele 11 - 15 werden analog zur Vorschrift des Beispiels 10 ausgehend von 100 mg (0.46 mmol) 5-Amino-1-(2-methylphenyl)-1H-pyrazol-4-carbonsäureamid (Beispiel 25A) mit 157 mg (2.31 mmol) Natriumethylat und mit 2.08 mmol des in der Tabelle aufgeführten Esters hergestellt. Das Rohprodukt wird jeweils über präparative HPLC aufgereinigt.

**Tabelle 1:**

| **Bsp. Nr.** | **Struktur** | **Edukte** | **Ausb.** **[%]** | **Rₜ [min]** **(Methode)** | **MS: m/z** **[M+H]⁺** |
|---|---|---|---|---|---|
| **11** | 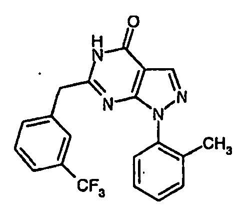 | Beispiel 25A, (3-Trifluormethylphenyl)-essigsäuremethylester | 60.1 | 2.04 (4) | 385 |
| **12** | 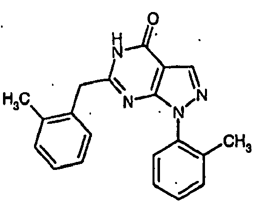 | Beispiel 25A, (2-Methyl-phenyl)-essigsäuremethylester | 43.9 | 2.00 (4) | 331 |
| **13** | 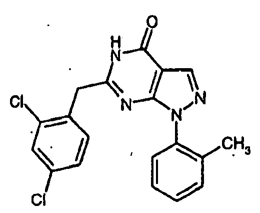 | Beispiel 25A, (2,4-Dichlorphenyl)-essigsäuremethylester | 39.2 | 2.15 (4) | 386 |
| **14** | 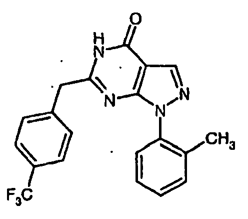 | Beispiel 25A, (4-Trifluormethylphenyl)-essigsäuremethylester | 12.0 | 2.05 (4) | 385 |
| **15** | 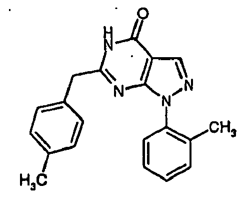 | Beispiel 25A, (4-Methylphenyl)-essigsäuremethylester | 34.8 | 1.98 (4) | 331 |

### Beispiel 16

### 6-(3-Chlorbenzyl)-1-(2-methylphenyl)-1,5-dihydro-4H-pyrazolo[3,4-d]pyrimidin-4-on

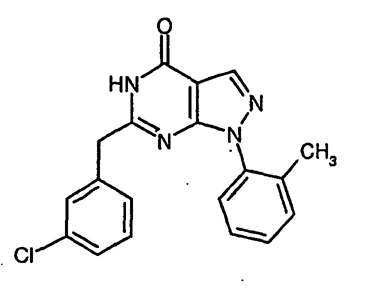

Analog zur Herstellung von Beispiel 9 werden ausgehend von 0.15 g (0.69 mmol) 5-Amino-1-(2-methylphenyl)-1H-pyrazol-4-carbonsäureamid (Beispiel 25A), 0.482 g (2.43 mol) (3-Chlorphenyl)essigsäureethylester und 0.139 g (3.47 mmol) 60%-igem Natriumhydrid 146 mg (60% d.Th.) des gewünschten Produktes als farbloser Feststoff erhalten.

Fp.: 215°C

MS (ESI pos): m/z = 351 (M+H)⁺

¹H-NMR (300 MHz, DMSO-d₆): δ = 2.05 (s, 3H), 3.9 (s, 2H), 7.2-7.5 (m, 8H), 8.25 (s, 1H), 12.5 (s, 1H) ppm.

### Beispiel 17

### 1-(2-Methylphenyl)-6-(2-pyridinylmethyl)-1,5-dihydro-4H-pyrazolo[3,4-d]pyrimidin-4-on

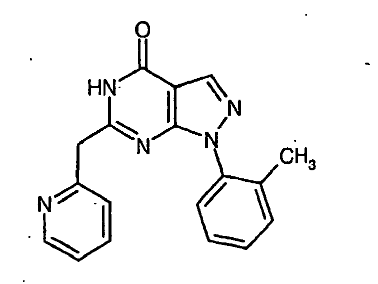

Analog zur Herstellung von Beispiel 9 werden ausgehend von 0.12 g (0.55 mmol) 5-Amino-1-(2-methylphenyl)-1H-pyrazol-4-carbonsäureamid (Beispiel 25A), 0.252 g (1.66 mmol) (2-Pyridinyl)-essigsäureethylester und 0.111 g (2.77 mmol) 60%-igem Natriumhydrid 71 mg (40% d.Th.) des gewünschten Produktes als farbloser Feststoff erhalten.

Fp.: 162°C

MS (ESI pos): m/z = 318 (M+H)⁺

¹H-NMR (300 MHz, DMSO-d₆): δ = 2.0 (s, 3H), 4.2 (s, 2H), 7.2-7.5 (m, 6H), 7.8 (t, 1H), 8.25 (s, 1H), 8.5 (d, 1H), 12.4 (s, 1H) ppm.

### Beispiel 18

### 6-(3-Chlorbenzyl)-1-(2-ethylphenyl)-1,5-dihydro-4H-pyrazolo[3,4-d]pyrimidin-4-on

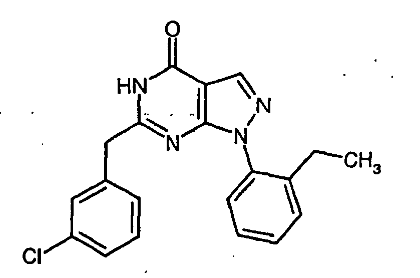

Analog zur Herstellung von Beispiel 9 werden ausgehend von 0.15 g (0.65 mol) 5-Amino-1-(2-ethylphenyl)-1H-pyrazol-4-carbonsäureamid (Beispiel 26A), 0.398 g (1.95 mmol) (3-Chlorphenyl)essigsäureethylester und 0.130 g (3.26 mmol) 60%-igem Natriumhydrid 65 mg (27% d.Th.) des gewünschten Produktes als farbloser Feststoff erhalten.

Fp.: 208°C

MS (ESI pos): m/z = 365 (M+H)⁺

¹H-NMR (300 MHz, DMSO-d₆): δ = 0.9 (t, 3H), 2.35 (q, 2H), 3.9 (s, 2H), 7.15-7.5 (m, 8H), 8.25 (s, 1H), 12.45 (s, 1H) ppm.

### Beispiel 19

### 6-(3-Chlorbenzyl)-1-(2-trifluormethylphenyl)-1,5-dihydro-4H-pyrazolo[3,4-d]pyrimidin-4-on

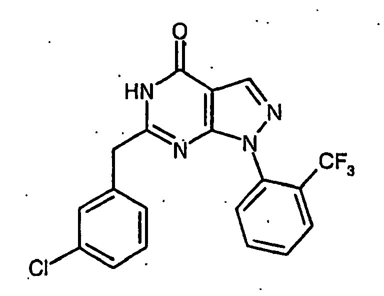

Analog zur Herstellung von Beispiel 9 werden ausgehend von 0.15 g (0.56 mmol) 5-Amino-1-(2-trifluormethylphenyl)-1H-pyrazol-4-carbonsäureamid (Beispiel 27A), 0.339 g (1.67 mmol) (3-Chlorphenyl)essigsäureethylester und 0.111 g (2.78 mmol) 60%-igem Natriumhydrid 157 mg (70% d.Th.) des gewünschten Produktes als farbloser Feststoff erhalten.

Fp.: 152°C

MS (ESI pos): m/z = 405 (M+H)⁺

¹H-NMR (300 MHz, DMSO-d₆): δ = 3.9 (s, 2H), 7.15-7.5 (m, 4H), 7.6-8.05 (m, 4H), 8.3 (s, 1H), 12.5 (s, 1H) ppm.

### Beispiel 20

### 6-(3-Chlorbenzyl)-1-(2-fluorphenyl)-1,5-dihydro-4H-pyrazolo[3,4-d]pyrimidin-4-on

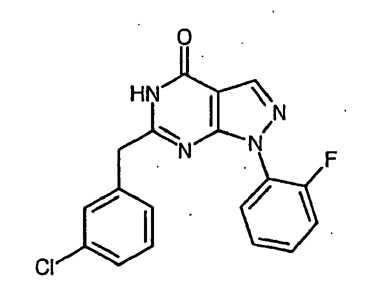

Analog zur Herstellung von Beispiel 9 werden ausgehend von 0.15 g (0.66 mmol) 5-Amino-1-(2-fluorphenyl)-1H-pyrazol-4-carbonsäureamid (Beispiel 28A), 0.405 g (98% Reinheit, 1.99 mmol) (3-Chlorphenyl)essigsäureethylester und 0.132 g (3.32 mmol) 60%-igem Natriumhydrid 171 mg (73% d.Th.) des gewünschten Produktes als farbloser Feststoff erhalten.

Fp.: 197°C

MS (ESI pos): m/z = 355 (M+H)^{+ 1}H-NMR (300 MHz, DMSO-d₆): δ = 3.95 (s, 2H). 7.2-7.7 (m, 8H), 8.3 (s, 1H), 12.5 (s, 1H) ppm.

### Beispiel 21

### 6-(3-Chlorbenzyl)-1-(2-chlorphenyl)-1,5-dihydro-4H-pyrazolo[3,4-d]pyrimidin-4-on

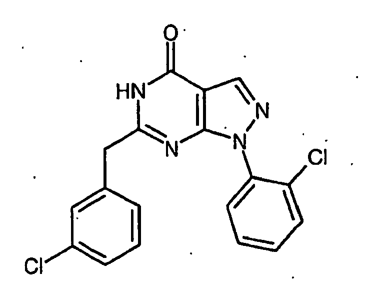

Analog zur Herstellung von Beispiel 9 werden ausgehend von 0.15 g (0.63 mmol) 5-Amino-1-(2-chlorphenyl)-1H-pyrazol-4-carbonsäureamid (Beispiel 29A), 0.387 g (98% Reinheit, 1.90 mmol) (3-Chlorphenyl)essigsäureethylester und 0.127 g (3.17 mmol) 60%-igem Natriumhydrid 160 mg (70% d.Th.) des gewünschten Produktes als farbloser Feststoff erhalten.

Fp.: 188°C

MS (ESI pos): m/z = 372 (M+H)⁺

¹H-NMR (300 MHz, DMSO-d₆): δ = 3.9 (s, 2H), 7.2-7.75 (m, 8H), 8.3 (s, 1H), 12.5 (s, 1H) ppm

### Beispiel 22

### 6-(3-Chlorbenzyl)-1-(2-pyridinyl)-1,5-dihydro-4H-pyrazolo[3,4-d]pyrimidin-4-on

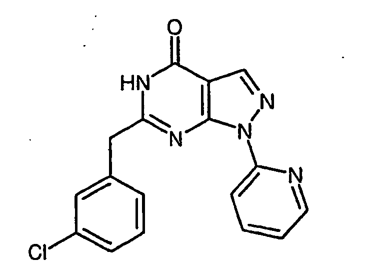

Analog zur Herstellung von Beispiel 9 werden ausgehend von 0.15 g (0.74 mmol) 5-Amino-1-(2-pyridinyl)-1H-pyrazol-4-carbonsäureamid (Beispiel 30A), 0.451 g (98% Reinheit, 2.21 mmol) (3-Chlorphenyl)essigsäureethylester und 0.148 g (3.69 mmol) 60%-igem Natriumhydrid 103 mg (41 % d.Th.) des gewünschten Produktes als farbloser Feststoff erhalten.

Fp.:230°C

MS (ESI pos): m/z = 338 (M+H)⁺

¹H-NMR (300 MHz, DMSO-d₆): δ = 4.0 (s, 2H), 7.2-7.75 (m, 5H), 7.9-8.05 (m, 2H), 8.3 (s, 1H), 8.6 (d, 1H), 12.5 (s, 1H) ppm.

### Beispiel 23

### 6-(3-Chlorbenzyl)-1-(2-methoxyphenyl)-1,5-dihydro-4H-pyrazolo[3,4-d]pyrimidin-4-on

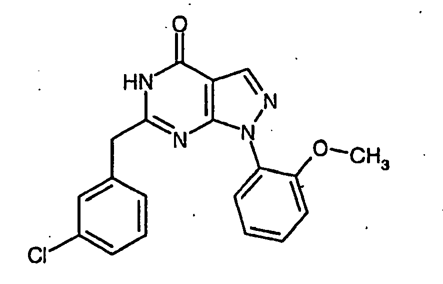

Analog zur Herstellung von Beispiel 9 werden ausgehend von 0.15 g (0.65 mmol) 5-Amino-1-(2-methoxyphenyl)-1H-pyrazol-4-carbonsäureamid (Beispiel 31A), 0.394 g (98% Reinheit, 1.94 mmol) (3-Chlorphenyl)essigsäureethylester und 0.129 g (3.23 mmol) 60%-igem Natriumhydrid 180 mg (76% d.Th.) des gewünschten Produktes als farbloser Feststoff erhalten.

Fp.: 196°C

MS (ESI pos): m/z = 367 (M+H)⁺

¹H-NMR (300 MHz, DMSO-d₆): δ = 3.7 (s, 3H), 3.9 (s, 2H), 7.0-7.6 (m, 8H), 8.2 (s, 1H), 12.4 (s, 1H) ppm.

## Patentansprüche

1. Verbindungen der Formel 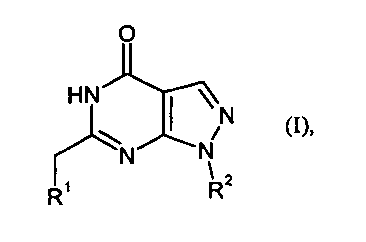 in welcher
R¹ Phenyl, Pyridyl oder Thiophenyl, welche gegebenenfalls mit bis zu 3 Substituenten unabhängig voneinander ausgewählt aus der Gruppe C₁-C₆-Alkyl, C₁-C₆-Alkoxy, Hydroxycarbonyl, Cyano, Trifluormethyl, Amino, Nitro, Hydroxy, C₁-C₆- Alkylamino, Halogen, C₆-C₁₀-Arylcarbonylamino, C₁-C₆-Alkylcarbonylamino, C₁-C₆- Alkylaminocarbonyl, C₁-C₆-Alkoxycarbonyl, C₆-C₁₀-Arylaminocarbonyl, Hetero- arylaminocarbonyl, Heteroarylcarbonylamino, C₁-C₆-Alkylsulfonylamino, C₁-C₆- Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆- Alkoxycarbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8-gliedriges Heterocyclyl bedeuten,
substituiert sind,
R² Phenyl oder Heteroaryl, wobei Phenyl mit 1 bis 3 Resten und Heteroaryl gegebenenfalls mit 1 bis 3 Resten jeweils unabhängig voneinander ausgewählt aus der Gruppe C₁-C₆-Alkyl, C₁-C₆-Alkoxy, Hydroxycarbonyl, Cyano, Trifluormethyl, Amino, Nitro, Hydroxy, C₁-C₆-Alkylamino, Halogen, C₆-C₁₀-Arylcarbonylamino, C₁- C₆-Alkylcarbonylamino, C₁-C₆-Alkylaminocarbonyl, C₁-C₆-Alkoxycarbonyl, C₆-C₁₀- Arylaminocarbonyl, Heteroarylaminocarbonyl, Heteroarylcarbonylamino, C₁-C₆- Alkylsulfonylamino, C₁-C₆-Alkylsulfonyl, C₁-C₆-Alkylthio substituiert sind,
wobei C₁-C₆-Alkyl, C₁-C₆-Alkoxy, C₁-C₆-Alkylamino, C₆-C₁₀-Arylcarbonylamino, C₁-C₆-Alkylcarbonylamino, C₁-C₆-Alkylaminocarbonyl, C₁-C₆-Alkoxy- carbonyl, C₆-C₁₀-Arylaminocarbonyl, Heteroarylaminocarbonyl, Heteroarylcarbonylamino, C₁-C₆-Alkylsulfonylamino, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei R³ und R⁴ die oben angegebenen Bedeutungen aufweisen,
substituiert sind,
bedeuten, sowie deren Salze, Solvate und/oder Solvate der Salze.

2. Verbindungen nach Anspruch 1, wobei
R¹ Phenyl, Pyridyl oder Thiophenyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkylcarbonylamino, C₁-C₄- Alkylaminocarbonyl, C₁-C₄-Alkoxycarbonyl, C₆-C₁₀-Arylaminocarbonyl, Heteroaryl- aminocarbonyl, Heteroarylcarbonylamino, C₁-C₄-Alkylsulfonylamino, C₁-C₄- Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ unabhängig voneinander Wasserstoff oder C₁-C₄-Alkyl, oder
R³ und R⁴ zusammen mit dem Stickstoffatom, an das sie gebunden sind, 5- bis 6-gliedriges Heterocyclyl bedeuten,
substituiert sind,
R² Phenyl, Pyrimidyl oder Pyridyl, wobei Phenyl mit 1 bis 3 Resten und Pyrimidyl und Pyridyl gegebenenfalls mit 1 bis 3 Resten jeweils unabhängig voneinander ausgewählt aus der Gruppe C₁-C₄-Alkyl, C₁-C₄-Alkoxy, Hydroxycarbonyl, Cyano, Trifluormethyl, Amino, Hydroxy, C₁-C₄-Alkylamino, Fluor, Chlor, Brom, C₆-C₁₀-Arylcarbonylamino, C₁-C₄-Alkylcarbonylamino, C₁-C₄-Alkylaminocarbonyl, C₁-C₄-Alkoxycarbonyl, C₆- C₁₀-Arylaminocarbonyl, Heteroarylaminocarbonyl, Heteroarylcarbonylamino, C₁-C₄- Alkylsulfonylamino, C₁-C₄-Alkylsulfonyl, C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei R³ und R⁴ die in Anspruch 1 angegebenen Bedeutungen aufweisen,
substituiert sind, bedeuten, sowie deren Salze, Solvate und/oder Solvate der Salze.

3. Verbindungen nach Ansprüchen 1 und 2, wobei R¹ die in Ansprüchen 1 und 2 angegebenen Bedeutungen aufweist und
R² Phenyl oder Pyridyl, wobei Phenyl mit 1 bis 2 Resten und Pyridyl gegebenenfalls mit 1 bis 2 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeutet, sowie deren Salze, Solvate und/oder Solvate der Salze.

4. Verbindungen nach Ansprüchen 1, 2 und 3, wobei
R¹ Phenyl, Pyridyl oder Thiophenyl, welche gegebenenfalls mit bis zu 2 Resten unabhängig voneinander ausgewählt aus der Gruppe C₁-C₄-Alkyl, Fluor, Chlor, Trifluormethyl, Hydroxy, Phenylcarbonylamino, C₁-C₄-Alkylcarbonylamino, C₁-C₄- Alkylaminocarbonyl oder Phenylaminocarbonyl substituiert sind,
R² Phenyl oder Pyridyl, wobei Phenyl mit 1 bis 2 Resten und Pyridyl gegebenenfalls mit 1 bis 2 Resten jeweils unabhängig voneinander ausgewählt aus der Gruppe Methyl, Ethyl, 2-Propyl, Trifluormethyl, Methoxy, Ethoxy, Fluor und Chlor substituiert sind,
bedeuten, sowie deren Salze, Solvate und/oder Solvate der Salze.

5. Verfahren zur Herstellung von Verbindungen nach Anspruch 1, **dadurch gekennzeichnet, dass** man
[A] Verbindungen der Formel 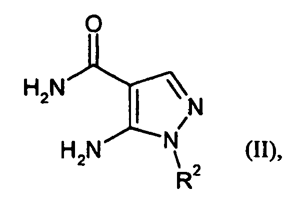 in welcher
R² die in Anspruch 1 angegebenen Bedeutungen hat,
durch Umsetzung mit einer Verbindung der Formel
R¹-CH₂-C(O)-Z (IIIa),
in welcher
R¹ die in Anspruch 1 angegebenen Bedeutungen hat,
und
Z für Chlor oder Brom steht,
in einem inerten Lösemittel und in Anwesenheit einer Base zunächst in Verbindungen der Formel 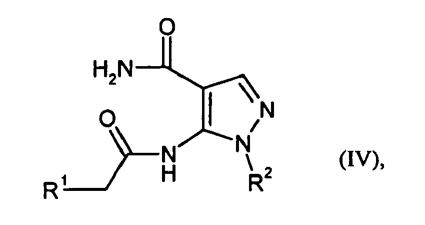 in welcher
R¹ und R² die in Anspruch 1 angegebenen Bedeutungen haben,
überführt, dann in einem inerten Lösemittel in Gegenwart einer Base zu Verbindungen der Formel (I) cyclisiert,
oder
[B] Verbindungen der Formel (II) unter direkter Cyclisierung zu (I) mit einer Verbindung der Formel
R¹-CH₂-C(O)-OR³ (IIIb),
in welcher
R¹ die in Anspruch 1 angegebenen Bedeutungen hat,
und
R³ für Methyl oder Ethyl steht,
in einem inerten Lösemittel und in Anwesenheit einer Base umsetzt, oder
[C] Verbindungen der Formel 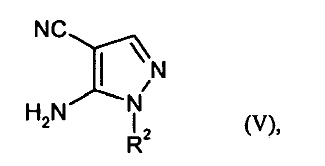 in welcher
R² die in Anspruch 1 angegebenen Bedeutungen hat,
zunächst durch Umsetzung mit einer Verbindung der Formel (IIIa) in einem inerten Lösemittel und in Anwesenheit einer Base in Verbindungen der Formel 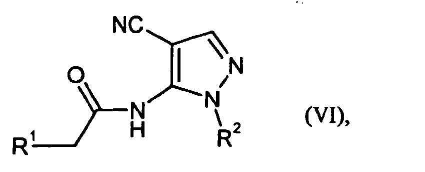 in welcher
R¹ und R² die in Anspruch 1 angegebenen Bedeutungen haben,
überführt,
und diese in einem zweiten Schritt in einem inerten Lösemittel und in Anwesenheit einer Base und eines Oxidationsmittels zu (I) cyclisiert,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

6. Verbindungen der Formel (I) nach einem der Ansprüche 1 bis 4 sowie deren physiologisch unbedenklichen Salze, Solvate und/oder Solvate dieser Salze zur Behandlung und/oder Prophylaxe von Krankheiten.

7. Arzneimittel enthaltend mindestens eine Verbindungen ausgewählt aus der Gruppe von Verbindungen der Formel (I) nach einem der Ansprüche 1 bis 4 sowie deren physiologisch unbedenklichen Salze, Solvate und/oder Solvate dieser Salze und mindestens einen pharmazeutisch verträglichen, im wesentlichen nichtgiftigen Träger oder Exzipienten.

8. Verwendung der Verbindungen der Formel (I) nach einem der Ansprüche 1 bis 4 sowie deren physiologisch unbedenklichen Salze, Solvate und/oder Solvate dieser Salze zur Herstellung eines Arzneimittels zur Prophylaxe und/oder Behandlung von Störungen der Wahrnehmung, Konzentrationsleistung, Lern- und/oder Gedächtnisleistung.

9. Verwendung nach Anspruch 8, wobei die Störung eine Folge der Alzheimer'schen Krankheit ist.

10. Verwendung der Verbindungen der Formel (I) nach einem der Ansprüche 1 bis 4 sowie deren physiologisch unbedenklichen Salze, Solvate und/oder Solvate dieser Salze zur Herstellung eines Arzneimittels zur Verbesserung der Wahrnehmung, Konzentrationsleistung, Lern-und/oder Gedächtnisleistung.

## Claims

1. Compounds of formula 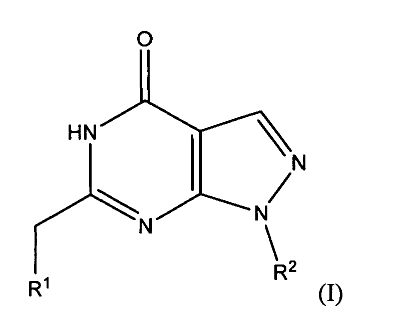 wherein
R¹ denotes phenyl, pyridyl or thiophenyl which may optionally be substituted by up to 3 substituents selected independently of one another from among C₁-C₆- alkyl, C₁-C₆-alkoxy, hydroxycarbonyl, cyano, trifluoromethyl, amino, nitro, hydroxy, C₁-C₆-alkylamino, halogen, C₆-C₁₀-arylcarbonylamino, C₁-C₆- alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆-alkylsulphonylamino, C₁-C₆-alkylsulphonyl and C₁-C₆-alkylthio,
wherein C₁-C₆-alkyl, C₁-C₆-alkoxy, C₁-C₆-alkylamino, C₆-C₁₀- arylcarbonylamino, C₁-C₆-alkylcarbonylainino, C₁-C₆- alkylaminocarbonyl, C₁-C₆-alkoxycarbonyl, C₆-C₁₀- arylaminocarbonyl, heteroarylaminocarbonyl, heteroarylcarbonylamino, C₁-C₆-alkylsulphonylamino, C₁-C₆- alkylsulphonyl and C₁-C₆-alkylthio are optionally substituted by a group selected from among hydroxy, cyano, halogen, hydroxycarbonyl and a group of the formula -NR³R⁴,
wherein
R³ and R⁴ independently of one another denote hydrogen or C₁-C₆-alkyl,
or
R³ and R⁴ together with the nitrogen atom to which they are bound denote 5- to 8-membered heterocyclyl,
R² R³ and R⁴ together with the nitrogen atom to which they are bound denote 5- to 8-membered heterocyclyl, denotes phenyl or heteroaryl, wherein phenyl is substituted by 1 to 3 groups and heteroaryl is optionally substituted by 1 to 3 groups selected independently of one another from among C₁-C₆-alkyl, C₁-C₆-alkoxy, hydroxycarbonyl, cyano, trifluoromethyl, amino, nitro, hydroxy, C₁-C₆- alkylamino, halogen, C₆-C₁₀-arylcarbonylamino, C₁-C₆-alkylcarbonylamino, C₁-C₆-alkylaminocarbonyl, C₁-C₆-alkoxycarbonyl, C₆-C₁₀-arylaminocarbonyl, heteroarylaminocarbonyl, heteroarylcarbonylamino, C₁-C₆- alkylsulphonylamino, C₁-C₆-alkylsulphonyl and C₁-C₆-alkylthio,
wherein C₁-C₆-alkyl, C₁-C₆-alkoxy, C₁-C₆-alkylamino, C₆-C₁₀- arylcarbonylamino, C₁-C₆-alkylcarbonylamino, C₁-C₆- alkylaminocarbonyl, C₁-C₆-alkoxycarbonyl, C₆-C₁₀- arylaminocarbonyl, heteroarylaminocarbonyl, heteroarylcarbonylamino, C₁-C₆-alkylsulphonylamino, C₁-C₆- alkylsulphonyl and C₁-C₆-alkylthio are optionally substituted by a group selected from among hydroxy, cyano, halogen, hydroxycarbonyl and a group of the formula -NR³R⁴,
wherein R³ and R⁴ are as hereinbefore defined,
and the pharmaceutically acceptable salts, solvates and/or solvates of the salts thereof.

2. Compounds according to claim 1, wherein
R¹ denotes phenyl, pyridyl or thiophenyl which may optionally be substituted by up to 3 groups selected independently of one another from among C₁-C₄- alkyl, C₁-C₄-alkoxy, hydroxycarbonyl, cyano, trifluoromethyl, amino, hydroxy, C₁-C₄-alkylamino, fluorine, chlorine, bromine, C₆-C₁₀- arylcarbonylamino, C₁-C₄-alkylcarbonylamino, C₁-C₄-alkylaminocarbonyl, C₁-C₄-alkoxycarbonyl, C₆-C₁₀-arylaminocarbonyl, heteroarylaminocarbonyl, heteroarylcarbonylamino, C₁-C₄-alkylsulphonylamino, C₁-C₄-alkylsulphonyl and C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of the formula -NR³R⁴,
wherein
R³ and R⁴ independently of one another denote hydrogen or C₁-C₄- alkyl,
or
R³ and R⁴ together with the nitrogen atom to which they are bound denote 5- to 6-membered heterocyclyl,
R² denotes phenyl, pyrimidyl or pyridyl, wherein phenyl is substituted by 1 to 3 groups and pyrimidyl and pyridyl are optionally substituted by 1 to 3 groups selected independently of one another from among C₁-C₄-alkyl, C₁-C₄- alkoxy, hydroxycarbonyl, cyano, trifluoromethyl, amino, hydroxy, C₁-C₄- alkylamino, fluorine, chlorine, bromine, C₆-C₁₀-arylcarbonylamino, C₁-C₄- alkylcarbonylamino, C₁-C₄-alkylaminocarbonyl, C₁-C₄-alkoxycarbonyl, C₆- C₁₀-arylaminocarbonyl, heteroarylaminocarbonyl, heteroarylcarbonylamino, C₁-C₄-alkylsulphonylamino, C₁-C₄-alkylsulphonyl and C₁-C₄-alkylthio,
wherein C₁-C₄-alkyl and C₁-C₄-alkoxy are optionally substituted by a group selected from among hydroxy, cyano, fluorine, chlorine, bromine, hydroxycarbonyl and a group of the formula -NR³R⁴,
wherein R³ and R⁴ have the meanings given in claim 1,
and the salts, solvates and/or solvates of the salts thereof.

3. Compounds according to claims 1 and 2, wherein R¹ has the meanings given in claims 1 and 2 and
R² denotes phenyl or pyridyl, wherein phenyl is substituted by 1 to 2 groups and pyridyl is optionally substituted by 1 to 2 groups selected independently of one another from among methyl, ethyl, 2-propyl, trifluoromethyl, methoxy, ethoxy, fluorine and chlorine,
and the salts, solvates and/or solvates of the salts thereof.

4. Compounds according to claims 1, 2 and 3, wherein
R¹ denotes phenyl, pyridyl or thiophenyl which may optionally be substituted by up to 2 groups selected independently of one another from among C₁-C₄-alkyl, fluorine, chlorine, trifluoromethyl, hydroxy, phenylcarbonylamino, C₁-C₄- alkylcarbonylamino, C₁-C₄-alkylaminocarbonyl or phenylaminocarbonyl,
R² denotes phenyl or pyridyl, wherein phenyl is substituted by 1 to 2 groups and pyridyl is optionally substituted by 1 to 2 groups selected independently of one another from among methyl, ethyl, 2-propyl, trifluoromethyl, methoxy, ethoxy, fluorine and chlorine,
and the salts, solvates and/or solvates of the salts thereof.

5. Process for preparing compounds according to claim 1, **characterised in that**
[A] compounds of formula 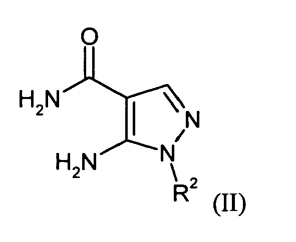 wherein
R² has the meanings given in claim 1,
are first converted by reaction with a compound of formula
R¹-CH₂-C(O)-Z (IIIa)
wherein
R¹ has the meanings given in claim 1,
and
Z denotes chlorine or bromine,
in an inert solvent and in the presence of a base, into compounds of formula 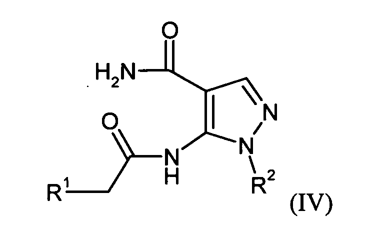 wherein
R¹ and R² have the meanings given in claim 1,
which are then cyclised in an inert solvent in the presence of a base to form compounds of formula (I),
or
[B] compounds of formula (II), while being directly cyclised to form (I), are reacted with a compound of formula
R¹-CH₂-C(O)-OR³ (IIIb)
wherein
R¹ has the meanings given in claim 1
and
R³ denotes methyl or ethyl,
in an inert solvent and in the presence of a base,
or
[C] compounds of the formula 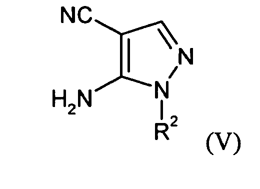 wherein
R² has the meanings given in claim 1,
is first converted by reaction with a compound of formula (IIIa), in an inert solvent and in the presence of a base, into compounds of formula 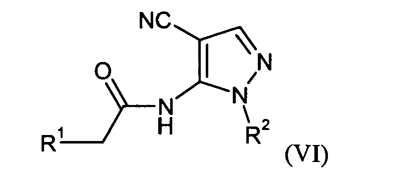 wherein
R¹ and R² have the meanings given in claim 1,
and these are then cyclised in a second step in an inert solvent and in the presence of a base and an oxidising agent to form (I),
and the resulting compounds of formula (I) are optionally reacted with the corresponding (i) solvents and/or (ii) bases or acids to form the solvates, salts and/or solvates of the salts thereof.

6. Compounds of formula (I) of one of claims 1 to 4 and the physiologically acceptable salts, solvates and/or solvates of these salts thereof, for the treatment and/or prevention of diseases.

7. Medicaments containing at least one compound selected from among the compounds of formula (I) of one of claims 1 to 4 and the physiologically acceptable salts, solvates and/or solvates of these salts thereof and at least one pharmaceutically acceptable, substantially non-toxic carrier or excipient.

8. Use of the compounds of formula (I) of one of claims 1 to 4 and the physiologically acceptable salts, solvates and/or solvates of these salts thereof for preparing a medicament for the prevention and/or treatment of disorders of cognition, concentration, learning and/or memory.

9. Use according to claim 8 wherein the disorder is a consequence of Alzheimer's disease.

10. Use of the compounds of formula (I) of one of claims 1 to 4 and the physiologically acceptable salts, solvates and/or solvates of these salts thereof for preparing a medicament for improving cognition, concentration, learning and/or memory.

## Revendications

1. Composés de formule 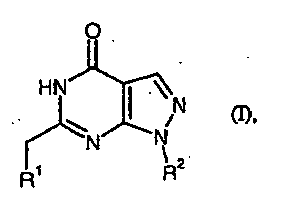 où
R¹ représente phényle, pyridyle ou thiophényle, qui sont éventuellement substitués avec jusqu'à 3 substituants choisis indépendamment les uns des autres dans le groupe C₁-C₆-alkyle, C₁-C₆-alcoxy, hydroxycarbonyle, cyano, trifluorométhyle, amino, nitro, hydroxy, C₁-C₆-alkylamino, halogène, C₆-C₁₀-arylcarbonylamino, C₁-C₆-alkylcarbonylamino, C₁-C₆-alkylaminocarbonyle, C₁-C₆-alcoxycarbonyle, C₆-C₁₀-arylaminocarbonyle, hétéroarylaminocarbonyle, hétéroarylcarbonylamino, C₁-C₆-alkylsulfonylamino, C₁-C₆-alkylsulfonyle, C₁-C₆-alkylthio,
où C₁-C₆-alkyle, C₁-C₆-alcoxy, C₁-C₆-alkylamino, C₆-C₁₀-arylcarbonylamino, C₁-C₆-alkylcarbonylamino, C₁-C₆-alkylaminocarbonyle, C₁-C₆-alcoxycarbonyle, C₆-C₁₀-arylaminocarbonyle, hétéroarylaminocarbonyle, hétéroarylcarbonylamino, C₁-C₆-alkylsulfonylamino, C₁-C₆-alkylsulfonyle et C₁-C₆-alkylthio sont éventuellement substitués avec un groupement choisi dans le groupe hydroxy, cyano, halogène, hydroxycarbonyle et un groupe de formule -NR³R⁴,
où
R³ et R⁴ représentent indépendamment l'un de l'autre l'hydrogène ou C₁-C₆-alkyle,
ou
R³ et R⁴ représentent avec l'atome d'azote auquel ils sont liés hétérocyclyle à 5 à 8 chaînons,
R² représente phényle ou hétéroaryle, où phényle est substitué avec 1 à 3 groupements et hétéroaryle est éventuellement substitué avec 1 à 3 groupements choisis chacun indépendamment des autres dans le groupe C₁-C₆-alkyle, C₁-C₆-alcoxy, hydroxycarbonyle, cyano, trifluorométhyle, amino, nitro, hydroxy, C₁-C₆-alkylamino, halogène, C₆-C₁₀-arylcarbonylamino, C₁-C₆-alkylcarbonylamino, C₁-C₆-alkylaminocarbonyle, C₁-C₆-alcoxycarbonyle, C₆-C₁₀-arylaminocarbonyle, hétéroarylaminocarbonyle, hétéroarylcarbonylamino, C₁-C₆-alkylsulfonylamino, C₁-C₆-alkylsulfonyle, C₁-C₆-alkylthio,
où C₁-C₆-alkyle, C₁-C₆-alcoxy, C₁-C₆-alkylamino, C₆-C₁₀-arylcarbonylamino, C₁-C₆-alkylcarbonylamino, C₁-C₆-alkylaminocarbonyle, C₁-C₆-alcoxycarbonyle, C₆-C₁₀-arylaminocarbonyle, hétéroarylaminocarbonyle, hétéroarylcarbonylamino, C₁-C₆-alkylsulfonylamino, C₁-C₆-alkylsulfonyle et C₁-C₆-alkylthio sont éventuellement substitués avec un groupement choisi dans le groupe hydroxy, cyano, halogène, hydroxycarbonyle et un groupe de formule-NR³R⁴,
où R³ et R⁴ présentent les significations indiquées ci-dessus, ainsi que leurs sels pharmaceutiquement acceptables, solvates et/ou solvates des sels.

2. Composés selon la revendication 1 où
R¹ représente phényle, pyridyle ou thiophényle, qui sont éventuellement substitués avec jusqu'à 3 groupements choisis indépendamment les uns des autres dans le groupe C₁-C₄-alkyle, C₁-C₄-alcoxy, hydroxycarbonyle, cyano, trifluorométhyle, amino, hydroxy, C₁-C₄-alkylamino, fluor, chlore, brome, C₆-C₁₀-arylcarbonylamino, C₁-C₄-alkylcarbonylamino, C₁-C₄-alkylaminocarbonyle, C₁-C₄-alcoxycarbonyle, C₆-C₁₀-arylaminocarbonyle, hétéroarylaminocarbonyle, hétéroarylcarbonylamino, C₁-C₄-alkylsulfonylamino, C₁-C₄-alkylsulfonyle, C₁-C₄-alkylthio,
où C₁-C₄-alkyle et C₁-C₄-alcoxy sont éventuellement substitués avec un groupement choisi dans le groupe hydroxy, cyano, fluor, chlore, brome, hydroxycarbonyle et un groupe de formule -NR³R⁴,
où
R³ et R⁴ représentent indépendamment l'un de l'autre l'hydrogène ou C₁-C₄-alkyle,
ou
R³ et R⁴ représentent avec l'atome d'azote auquel ils sont liés hétérocyclyle à 5 à 6 chaînons,
R² représente phényle, pyrimidyle ou pyridyle, où phényle est substitué avec 1 à 3 groupements et pyrimidyle et pyridyle sont éventuellement substitués avec 1 à 3 groupements choisis chacun indépendamment des autres dans le groupe C₁-C₄-alkyle, C₁-C₄-alcoxy, hydroxycarbonyle, cyano, trifluorométhyle, amino, hydroxy, C₁-C₄-alkylamino, fluor, chlore, brome, C₆-C₁₀-arylcarbonylamino, C₁-C₄-alkylcarbonylamino, C₁-C₄-alkylaminocarbonyle, C₁-C₄-alcoxycarbonyle, C₆-C₁₀-arylaminocarbonyle, hétéroarylaminocarbonyle, hétéroarylcarbonylamino, C₁-C₄-alkylsulfonylamino, C₁-C₆-alkylsulfonyle, C₁-C₆-alkylthio,
où C₁-C₄-alkyle et C₁-C₄-alcoxy sont éventuellement substitués avec un groupement choisi dans le groupe hydroxy, cyano, fluor, chlore, brome, hydroxycarbonyle et un groupe de formule -NR³R⁴,
où R³ et R⁴ présentent les significations indiquées dans la revendication 1,
ainsi que leurs sels, solvates et/ou solvates des sels.

3. Composés selon les revendications 1 et 2 où R¹ présente les significations indiquées dans les revendications 1 et 2 et
R² représente phényle ou pyridyle, où phényle est substitué avec 1 à 2 groupements et pyridyle est éventuellement substitué avec 1 à 2 groupements choisis chacun indépendamment de l'autre dans le groupe méthyle, éthyle, 2-propyle, trifluorométhyle, méthoxy, éthoxy, fluor et chlore,
ainsi que leurs sels, solvates et/ou solvates des sels.

4. Composés selon les revendications 1, 2 et 3 où
R¹ représente phényle, pyridyle ou thiophényle, qui sont éventuellement substitués avec jusqu'à 2 groupements choisis indépendamment l'un de l'autre dans le groupe C₁-C₄-alkyle, fluor, chlore, trifluorométhyle, hydroxy, phénylcarbonylamino, C₁-C₄-alkylcarbonylamino, C₁-C₄-alkylaminocarbonyle et phénylaminocarbonyle,
R² représente phényle ou pyridyle, où phényle est substitué avec 1 à 2 groupements et pyridyle est éventuellement substitué avec 1 à 2 groupements choisis chacun indépendamment de l'autre dans le groupe méthyle, éthyle, 2-propyle, trifluorométhyle, méthoxy, éthoxy, fluor et chlore,
ainsi que leurs sels, solvates et/ou solvates des sels.

5. Procédé de production de composés selon la revendication 1 **caractérisé en ce que**
[A] on convertit des composés de formule 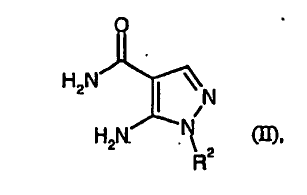 où
R² a les significations indiquées dans la revendication 1,
par réaction avec un composé de formule
R¹-CH₂-C(O)-Z (IIIa)
où
R¹ a les significations indiquées dans la revendication 1,
et
Z représente le chlore ou le brome,
dans un solvant inerte et en présence d'une base tout d'abord en composés de formule 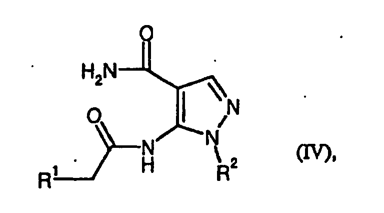 où
R¹ et R² ont les significations indiquées dans la revendication 1, puis, dans un solvant inerte en présence d'une base on cyclise en composés de formule (I),
ou
[B] on convertit des composés de formule (II) par cyclisation directe en (I) avec un composé de formule
R¹-CH₂-C(O)-OR³ (IIIb)
où
R¹ a les significations indiquées dans la revendication 1,
et
R³ représente méthyle ou éthyle,
dans un solvant inerte et en présence d'une base,
ou
[C] on convertit des composés de formule 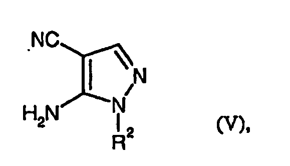 où
R² a les significations indiquées dans la revendication 1,
d'abord par réaction avec un composé de formule (IIIa) dans un solvant inerte et en présence d'une base en composés de formule 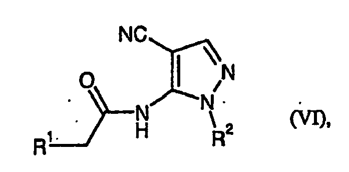 où
R¹ et R² ont les significations indiquées dans la revendication 1,
et on cyclise ceux-ci en (I) dans une deuxième étape dans un solvant inerte et en présence d'une base et d'un oxydant,
et on convertit éventuellement les composés de formule (I) résultants en leurs solvates, sels et/ou solvates des sels avec les (i) solvants et/ou (ii) bases ou acides correspondants.

6. Composés de formule (I) selon une des revendications 1 à 4 ainsi que leurs sels physiologiquement acceptables, solvates et/ou solvates de ces sels pour le traitement et/ou la prophylaxie des maladies.

7. Médicament contenant au moins un composé choisi dans les composés de formule (I) selon une des revendications 1 à 4 ainsi que leurs sels physiologiquement acceptables, solvates et/ou solvates de ces sels et au moins un vecteur ou excipient sensiblement non toxique, pharmaceutiquement acceptable.

8. Utilisation des composés de formule (I) selon une des revendications 1 à 4 ainsi que leurs sels physiologiquement acceptables, solvates et/ou solvates de ces sels pour la production d'un médicament pour la prophylaxie et/ou le traitement des troubles de la perception, des performances de concentration, des performances d'apprentissage et/ou de mémoire.

9. Utilisation selon la revendication 8 où le trouble est une conséquence de la maladie d'Alzheimer.

10. Utilisation des composés de formule (I) selon une des revendications 1 à 4 ainsi que leurs sels physiologiquement acceptables, solvates et/ou solvates de ces sels pour la production d'un médicament pour améliorer la perception, les performances de concentration, les performances d'apprentissage et/ou de mémoire.
